# EUROPEAN PATENT APPLICATION

(11) **EP 3 770 264 A1**
(43) Date of publication of application: **27.01.2021**
(21) Application number: 19305967.2
(22) Date of filing: 22.07.2019
(51) Int. Cl.: C12N 15/86, C12N 15/90, A61K 48/00, C12N 5/078, C07K 14/755

(54) **PRECISE INTEGRATION USING NUCLEASE TARGETED IDLV**

(71) Applicant: Genethon, 91000 Evry (FR); INSTITUT NATIONAL DE LA SANTE ET DE LA RECHERCHE MEDICALE - INSERM, 75013 Paris (FR); Universite d'Evry Val d'Essonne, 91000 Evry (FR)
(72) Inventor: AMENDOLA, Mario, 75011 PARIS (FR); PAVANI, Giulia, 75020 PARIS (FR); SAKKAL, Aboud, 91260 JUVISY SUR ORGE (FR)
(74) Representative: Nony

(57) **Abstract**

The present invention relates to an integration-defective lentiviral vector (IDLV) comprising a nucleic acid, the said nucleic acid comprising, between a 5' LTR sequence and a 3' LTR sequence, at least one nucleus export signaling sequence; at least one nucleic acid sequence of interest; at least one nuclease site, and at least one homology arm sequence. The invention further relates to an isolated cell comprising said IDLV, a pharmaceutical composition comprising said IDLV or said isolated cell, and their pharmaceutical use in the treatment of a disease selected from the group consisting of immune diseases, viral infections, tumors and blood diseases; and/or a disease caused by the lack of a protein or by the presence of an aberrant non-functional one in an individual in need thereof.

## Description

### FIELD OF THE INVENTION

The present invention relates to the field of genome engineering and DNA repair.

In particular, the invention relates to IDLV able to perform a precise and non-toxic sequence integration in the genome of a cell, with a high efficiency materialized by an efficient knock-in (KI).

### BACKGROUND OF THE INVENTION

One of the major therapeutic approaches to combat detrimental cellular phenotypes caused by loss of function mutations relies on intracellular delivery of a wild-type gene copy. In this regard, viral-mediated gene-replacement therapy, a rapidly evolving field, has provided some solutions limited by incomplete control over transgene copy numbers and expression levels; in addition, semi-random integration of lentiviral and retroviral vectors can result in insertional mutagenesis and activation of proto-oncogenes, while Adeno-associated vector (AAV) and adenoviral vectors deliver DNA as episome, which gets diluted in cycling cells.

In recent years, a new strategy has been developed that uses genomic DNA cleavage with site-specific nucleases to bias transgene integration into a chosen locus. One approach involves the integration of a transgene into its cognate locus, for example, insertion of a wild type transgene into the endogenous locus to correct a mutant gene. Alternatively, the transgene may be inserted into a non-cognate locus chosen specifically for its beneficial properties, such as permanent, safe, and very high levels of transgene expression.

While interesting, achieving efficient and non-toxic delivery and efficient site-specific integration of a transgene remains a complicated issue to solve.

Targeted genome editing with engineered nucleases is revolutionizing basic biomedical research and holds tremendous potential for gene therapy. However, and despite rapid advances, the sought-after goal of targeted transgene integration (knock-in, KI) for clinical purposes is still unsettled due to a lack of efficient tools. While KI in dividing and non-dividing cells has been already successfully described, the principal means of transgene delivery is plasmid DNA, which is toxic in primary cells (especially hematopoietic stem cells, HSC), does not allow targeting of specific cells and is not feasible for *in vivo* delivery.

Viral vector DNA delivery represents an interesting alternative to plasmid DNA, in particular AAV and integrase defective lentiviral vectors (IDLV). Lentivirus are + single stranded RNA (-9.7 Kb) and, once in the cell nucleus, are retrotranscribed to generate double stranded DNA, which is semi-randomly integrated in the host cellular genome. IDLV are derived from lentivirus by inactivating the integrase protein and thus blocking their ability to integrate in the genome. IDLV genome persists in the nucleus under different molecular forms before being lost. Unfortunately, IDLV represent an inefficient DNA substrate both for homologous (HDR) and non-homologous end-joining (NHEJ).

AAV are single stranded DNA virus (-4.7 kb) and, once in cell nucleus of target cells, become double stranded and circularize to persist as episome. AAV are a good substrate for HDR mediated KI; however, HDR is limited by its low efficiency in most primary cells and occurs mostly in the S/G2 phases of the cell cycle, making it inefficient in non-dividing cells. In addition, for HDR it is necessary to flank the transgene with genomic fragments homologous to the genomic DNA cutting site (∼800 bp each), which limits the size of the transgene that can be delivered by the AAV (packaging capacity of -4.7 kb), and can affect AAV production and transduction (for example in case of secondary structures). For this reason, about 6% of all human proteins, which have a coding sequence that exceeds 4kb, cannot fit in the AAV and expression elements, such as promoter and polyadenylation signal (polyA signal), need to be reduced in size, often limiting their transcriptional strength and tissue specificity (Chamberlain, Riyad and Weber, Hum. Gene Ther. Methods. 2016 Feb;27(1):1-12). Recently, homology-independent KI strategy was described to perform NHEJ based KI of AAV *in vivo* in brain and muscle; however, reported efficiency is very low, less than 5% in most cases, and the size of the DNA fragment integrated is very small, less than 700 bp (Suzuki et al., Nature. 2016 Dec 1;540(7631):144-149).

For KI in HSC, both IDLV and AAV containing homology arms for HDR mediated KI have been used. With IDLV, only very low efficiencies have been reported *ex vivo* and minimal upon transplantation in mice (<1% Kuo et al. Cell Rep. 2018 May 29;23(9):2606-2616; ∼5% Genovese et al. Nature. 2014 Jun 12;510(7504):235-240; almost undetectable, Schiroli et al., Sci Transl Med. 2017 Oct 11;9(411); <1% Hoban, Blood, 2015, Apr 23;125(17):2597-604). The AAV6 is more promising and the most recent reports show an *ex vivo* KI of 30-40%; however, AAV are also toxic for stem cells as they induce p53 activation and cell apoptosis (Hirsch, PlosOne, 2011; 6(11):e27520). AAV6 transduction shows some cellular toxicity (Kuo et al. Cell Rep. 2018 May 29;23(9):2606-2616) and reduce engraftment of modified hematopoietic stem cell (Schiroli et al., Cell Stem Cell, 2019 Apr 4;24(4):551-565). Moreover, HDR mediated KI occurs mostly in progenitor cells at the expenses of the real long-term HSC, which are targeted ∼5 times less (Gomez-Ospina et al., Human genome-edited hematopoietic stem cells phenotypically correct Mucopolysaccharidosis type I; Bioarchive; doi: https://doi.org/10.1101/408757). Finally, AAV delivery is affected by its packaging limitations, as described above.

Accordingly, as mentioned above, there is a need for an efficient, site-specific and non-toxic delivery method of a transgene in a cell. There is indeed a need for a novel tool allowing precise, efficient and non-toxic delivery of a sequence of interest in a cell. Said method and tool should in particular allow the delivery of a sequence of interest having a significant size variability, and in particular allow the delivery of sequences having a size of at least one kb (kilobase), and in particular of several kb.

The invention herein described has for purpose to meet the aforementioned needs.

### SUMMARY OF THE INVENTION

The present invention relates to an integration-defective lentiviral vector (IDLV) comprising a nucleic acid, the said nucleic acid comprising, between a 5' LTR sequence and a 3' LTR sequence:
a. at least one nucleus export signaling sequence, in particular at least one HIV-1 Rev Response Element (RRE), in particular one RRE,
b. at least one nucleic acid sequence of interest selected from the group consisting of a polyA signal; a splicing signal sequence; a DNA or RNA binding site; a promoter; and a transgene, or a fragment thereof, encoding a therapeutic protein or a therapeutic ribonucleic acid,
c. at least one nuclease site, in particular at least one guide nucleic acid targeted sequence (gRNA-T),
d. at least one homology arm sequence consisting in a sequence which is homologous to a part of an endogenous genomic site of interest in the genome of a cell; and
e. optionally, at least one sequence which allows enhancing stable expression of the at least one nucleic acid sequence of interest, in particular at least one Woodchuck hepatitis virus Post-transcriptional Regulatory Element (WPRE) sequence.

The inventors indeed managed to generate a novel IDLV able to perform a precise and non-toxic sequence integration in the genome of a cell, with a high efficiency materialized by an efficient KI, even with an integrated sequence having a substantial length (∼9kb).

According to a particular embodiment, the said IDLV is circular or linear, and is in particular circular.

According to an embodiment of the invention, the IDLV comprises at least two identical or different nuclease sites, in particular at least two identical or different gRNA-T sequences, and in particular two identical or different gRNA-T sequences. In particular, the at least one nucleic acid sequence of interest can be comprised between the at least two, and in particular two, nuclease sites of the said IDLV.

According to an embodiment of the invention, the at least one nuclease site of an IDLV of the invention is identical to, or different from, an endogenous nuclease site comprised in the endogenous genomic site of interest in the genome of the cell of point d.

In a particular embodiment, the at least one nucleic acid sequence of interest of an IDLV of the invention is a transgene, or a fragment thereof, encoding a therapeutic protein or a therapeutic ribonucleic acid.

In particular, the therapeutic protein can be selected from the group consisting of cytokines, in particular interferon, more particularly interferon-alpha, interferon-beta or interferon-pi; hormones; chemokines; antibodies (including nanobodies); anti-angiogenic factors; enzymes for replacement therapy, such as for example adenosine deaminase, alpha glucosidase, alpha-galactosidase, alpha-L-iduronidase and beta-glucosidase; interleukins; insulin; G-CSF; GM-CSF; hPG-CSF; M-CSF; blood clotting factors such as Factor VIII, Factor IX, tPA, Factor V, Factor VII, Factor X, Factor XI, Factor XII or Factor XIII; transmembrane proteins such as Nerve Growth Factor Receptor (NGFR); lysosomal enzymes such as α-galactosidase (GLA), α-L-iduronidase (IDUA), lysosomal acid lipase (LAL) and galactosamine (N-acetyl)-6-sulfatase (GALNS); beta-like globin; interleukin receptors such as IL-2 receptor, IL-3 receptor, IL-4 receptor, IL-5 receptor, IL-6 receptor, IL-7 receptor, IL-9 receptor, IL-11 receptor, IL-12 receptor, IL-13 receptor, IL-15 receptor, IL-21 receptor, IL-23 receptor and IL-27 receptor; Wiskott-Aldrich syndrome protein (WASP); adenosine deaminase, tripeptidyl peptidase 1, alpha-L iduronidase, iduronate 2-sulfatase, N-sulfoglucosamine sulfohydrolase, galactosamine-6 sulfatase, beta-galactosidase, N-acetylgalactosamine- 4-sulphatase, glucocerebrosidase, arylsulfatase A, cytochrome b-245 alpha chain, cytochrome b-245 beta chain, neutrophil cytosolic factor 1, neutrophil cytosolic factor 2, neutrophil cytosolic factor 4; any protein that can be engineered to be secreted and eventually uptaken by non-modified cells, and combinations thereof.

In an embodiment of the invention, the IDLV comprises at least two homology arm sequences, in particular two, each one being different from the other and consisting in sequences which are homologous to at least two, in particular two, different parts of an endogenous genomic site of interest in the genome of the cell.

In a particular embodiment, an IDLV of the invention does not comprise a promotor.

In an embodiment of the invention, the endogenous genomic site of interest in the genome of the cell is comprised within a globin gene, in particular selected from the group consisting of the epsilon globin gene, the gamma G globin gene, the gamma A globin gene, the delta globin gene, the beta globin gene, the zeta globin gene, the pseudozeta globin gene, the mu globin gene, the pseudoalpha-1 globin gene, the alpha 1 globin gene and the alpha 2 globin gene, in particular selected from the group consisting of the gamma G globin gene, the gamma A globin gene, the delta globin gene, the beta globin gene, the alpha 1 globin gene and the alpha 2 globin gene, more particularly selected from the group consisting of the alpha 1 globin gene and the alpha 2 globin gene.

In another embodiment, the sequences a to d, and e if present, of an IDLV of the invention are present in the IDLV, from 5' to 3', in one of the following orders:
- a, c, d, b;
- a, c, d, b, e;
- a, c, d, b, d, c;
- a, c, d, b, d, c, e;
- a, d, c, d, b;
- a, d, c, d, b, e;
- a, c, b, d, c, e; or
- a, c, d, b, c, e.

Another object of the present invention relates to an isolated cell comprising at least one IDLV of the invention.

In particular, the cell can be selected from the group consisting of hematopoietic stem cells; cells of the immune system, in particular lymphocytes; pluripotent stem cells; embryonic stem cells; satellite cells; neural stem cells; mesenchymal stem cells; retinal stem cells; and epithelial stem cells, and is in particular a hematopoietic stem cell.

A further object of the invention relates to a pharmaceutical composition comprising at least one IDLV of the invention and/or at least one isolated cell of the invention, and a pharmaceutically acceptable medium.

Another object of the present invention relates to an IDLV of the invention, an isolated cell of the invention or a pharmaceutical composition of the invention for use in the treatment of:
- a disease selected from the group consisting of immune diseases, viral infections, tumors and blood diseases; and/or
- a disease caused by the lack of a protein or by the presence of an aberrant non-functional one in an individual in need thereof.

A further object of the invention relates to a method for generating CAR-T cells comprising integrating, into a lymphocyte T cell or into a hematopoietic stem cell, at least one IDLV of the invention,
the said IDLV comprising, as at least one nucleic acid sequence of interest, a transgene encoding a chimeric antigen receptor targeting cancer cells.

Another object of the present invention relates to a method for generating antibody expressing B-cells, comprising integrating, into a lymphocyte B cell or into a hematopoietic stem cell, at least one IDLV of the invention,

the said IDLV comprising, as at least one nucleic acid sequence of interest, a transgene encoding an antibody.

### DESCRIPTION OF THE FIGURES

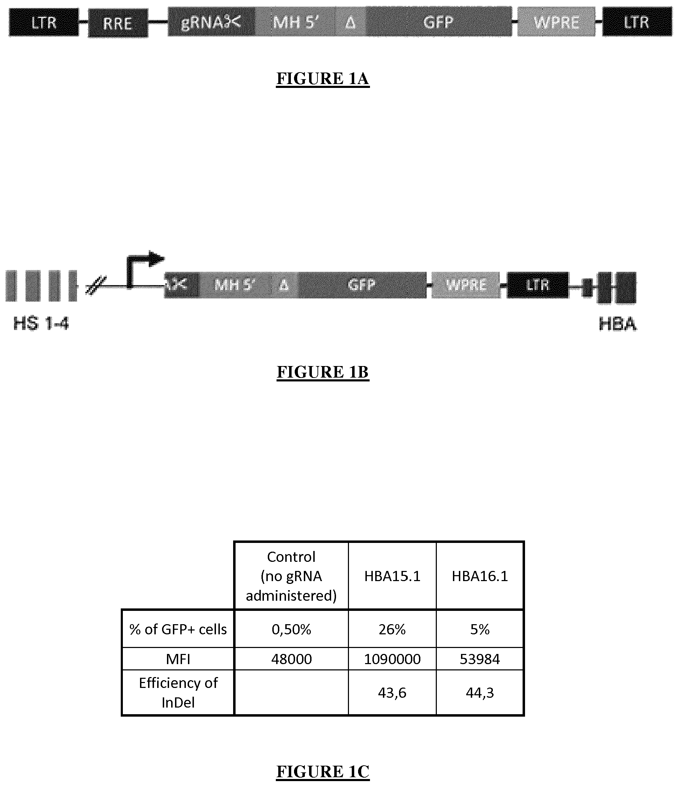

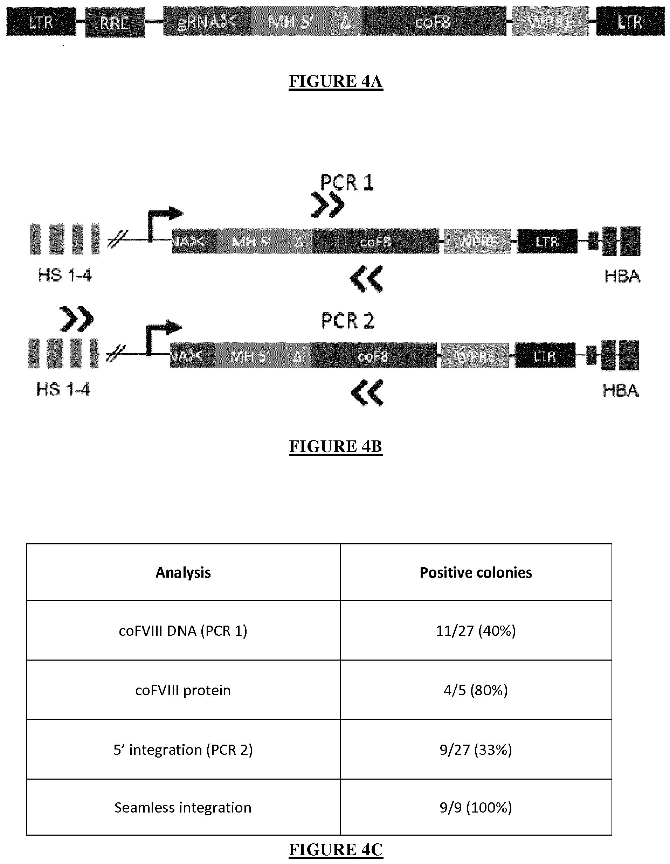

**Figure 1A****:** Schematic representation of an IDLV according to the invention comprising, between the Long terminal repeats (LTR) sequences, from left to right, a Rev Response Element (RRE), a gRNA cutting site (gRNA-T) (gRNA 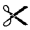 ) (nuclease site), 35 bp of microhomology (MH 5') (homology arm) identical to a part an endogenous genomic site of interest in the genome of the cell into which the IDLV will be integrated, a sequence encoding a promoterless (Δ) green fluorescent protein (GFP) and the Woodchuck Hepatitis Virus Posttranscriptional Regulatory Element (WPRE). This cassette is in sense orientation with respect to the LTR and RRE viral sequences.
**Figure 1B****:** Schematic representation of one possible outcome of IDLV integration in cell genome upon Cas9 cutting. Endogenous α-globin promoter (HS1-4) drives the expression of GFP, which is inserted between α-globin promoter and α gene (HBA).
**Figure 1C****:** Table shows the % of GFP+ cells and GFP MFI (median fluorescence intensity) values of gated cells measured by Flow cytometry analysis of GFP expression in K562-Cas9 cells (Human leukemic cell line K562 stably expressing Cas9) after GFP integration in the HBA gene using the IDLV described in point (A) and different gRNAs (HBA15.1 or HBA16.1). The efficiency of InDel is also measured for the two methods implementing a gRNA. The results obtained with a "no-gRNA" control are also shown.
**Figure 2A**: Schematic representation of the PCRs performed to analyze IDLV genomic integration in K562-Cas9 cell clones. The double arrows represent the primers used for the amplifications.
**Figure 2B****:** Summary table of DNA and FACS analyses of 163 clones analyzed. To establish if the integration was seamless, the inventors performed Sanger sequencing of each PCR product coming from PCR 2.
**Figure 3****:** K562 cells are transduced with the IDLV of the invention illustrated in Figure 1A and, after the amount of time indicated in the Table (4 hours, 8 hours, 24 hours, 32 hours or 48 hours), the cells are transfected with RNP (preassembled Cas9/gRNA complex) using different gRNAs (HBA15.1 or HBA16.1).
   Flow cytometry analysis of GFP expression is performed after 2 weeks from IDLV transduction, after a delay of 4 hours, 8 hours, 24 hours, 32 hours and 48 hours between IDLV and RNP delivery. A control is performed where RNP is not introduced.
   The Table shows the % of GFP+ cells and GFP MFI (median fluorescence intensity) values of gated cells. The efficiency of InDel is also measured for the two methods implementing the HBA15.1 gRNA or the HBA16.1 gRNA. The results obtained with the control are also shown
**Figure 4A****:** Schematic representation of an IDLV according to the invention comprising, between the Long terminal repeats (LTR) sequences, from left to right, a Rev Response Element (RRE), a gRNA cutting site (gRNA-T) (gRNA 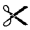 ) (nuclease site), 35 bp of microhomology (MH 5') (homology arm) identical to a part an endogenous genomic site of interest in the genome of the cell into which the IDLV will be integrated, a sequence coding for a promoterless (Δ) codon optimized Factor VIII (FVIII) (coF8) and the Woodchuck Hepatitis Virus Posttranscriptional Regulatory Element (WPRE). This cassette is in sense orientation with respect to the LTR and RRE viral sequences.
**Figure 4B****:** Schematic representation of the PCR performed to analyze IDLV genomic integration in K562-Cas9 (**Please confirm**) cell clones. The double arrows represent the primers used for the amplifications.
   It is also represented in this Figure the schematic of one possible outcome of IDLV integration in the genome of K562-Cas9 cells upon Cas9 cutting. Endogenous α-globin promoter (HS1-4) drives the expression of GFP, which is inserted between α-globin promoter and α gene (HBA).
**Figure 4C****:** Summary table of DNA and ELISA analyses of 27 clones analyzed. To establish if the integration was seamless, the inventors performed Sanger sequencing of each PCR product coming from PCR 2.

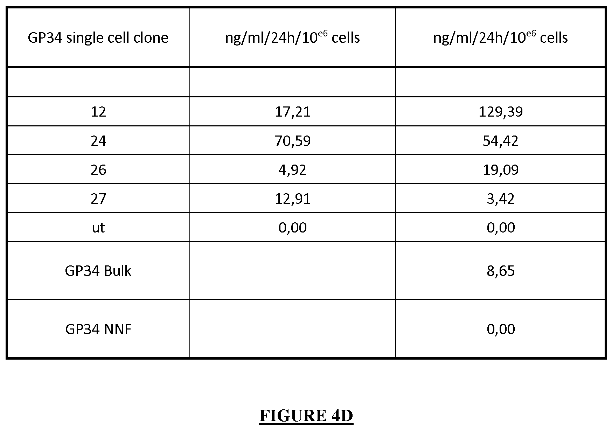

**Figure 4D****:** Results obtained following an Elisa for FVIII present in supernatants of bulk and single K562-Cas9 clones with targeted integration of the FVIII cassette, performed in duplicate (Tests 1 and 2).
   From top to bottom: Clone 12, Clone 24, Clone 26, Clone 27, ut (Untreated cells), Cells bulk (GP34 Bulk), NNF (Non-nucleofected cells - GP34 NNF).

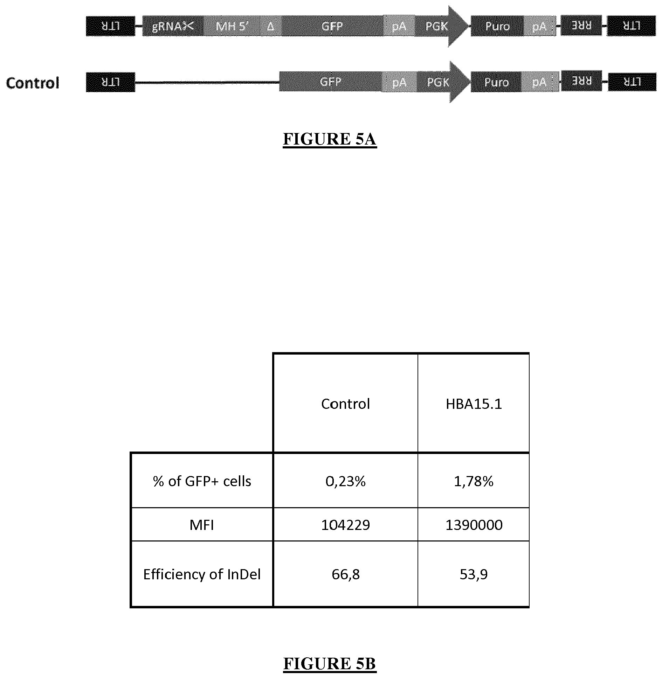

**Figure 5A****:** Schematic representation of an IDLV according to the invention comprising, between the Long terminal repeats (LTR) sequences, from left to right, a gRNA cutting site (gRNA-T) (gRNA 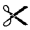 ) (nuclease site), 35 bp of microhomology (MH 5') (homology arm) identical to a part an endogenous genomic site of interest in the genome of the cell into which the IDLV will be integrated, a sequence encoding a promoterless (Δ) green fluorescent protein (GFP), a polyadenylation signal A (polyA signal or pA), the human Phosphoglycerate kinase (PGK) promoter driving the constitutive expression of the puromycin resistance gene (Puro) and a Rev Response Element (RRE). This cassette is in anti-sense orientation with respect to the LTR and RRE viral sequences.
   Right under this schematic representation is another schematic representation corresponding to a control cassette. This cassette differs from the one according to the invention defined above in that it does not comprise the cutting site (gRNA-T) (gRNA 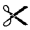 ) (nuclease site) and the 35 bp of the MH 5' homology arm sequences.
**Figure 5B****:** Table showing the % of GFP+ cells and GFP MFI (median fluorescence intensity) values of gated cells measured by Flow cytometry analysis of GFP expression in K562-Cas9 cells after GFP integration in the HBA gene of the K562-Cas9 cells using the IDLV described in point (A) and HBA15.1 gRNA. The efficiency of InDel is also measured. The results obtained with a "no-gRNA-T sequence" control are also shown.

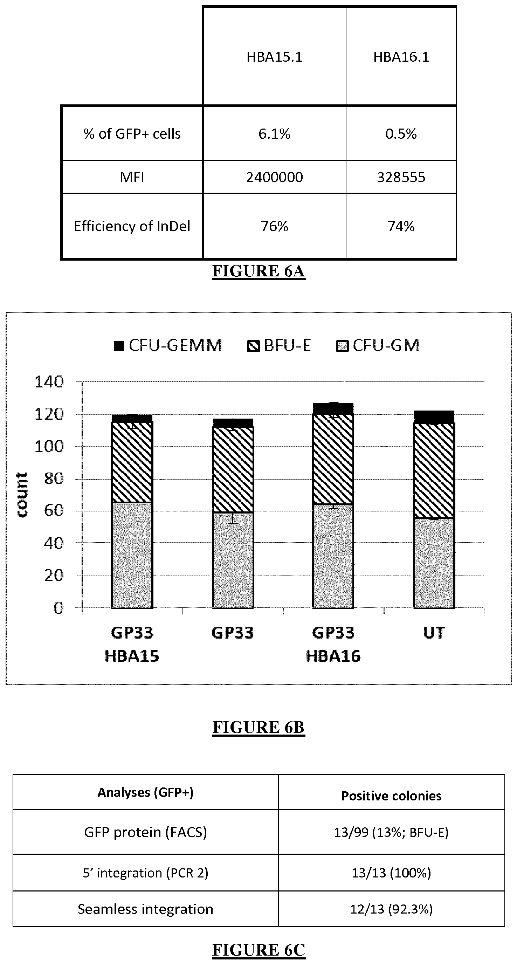

**Figure 6A****:** Table showing the % of GFP+ cells and GFP MFI (median fluorescence intensity) values of gated cells measured by Flow cytometry analysis of GFP expression in erythroid-differentiated primary HSPC after GFP integration in the HBA gene of the cells using the IDLV described in Figure 1A and different gRNAs (HBA15.1 or HBA16.1). The efficiency of InDel is also measured.
**Figure 6B****:** Colony-forming unit (CFU) assay of edited cells. Bars represent the average counts of two plates with standard deviation.
   Ordinate: count
   Abscissa: from left to right: Cells from Figure 6A with HBA15.1 (GP33 HBA15); Cells from Figure 6A without any gRNA (GP33); Cells from Figure 6A with HBA16.1 (GP33 HBA16), Untreated cells (UT).
   In each column, from the basis to the top: CFU-GEMM (granulocyte/macrophage forming units; BFU-E (erythroid burst-forming units) and CFU-GM (granulocyte/erythrocyte/monocyte/megakaryocyte forming units).

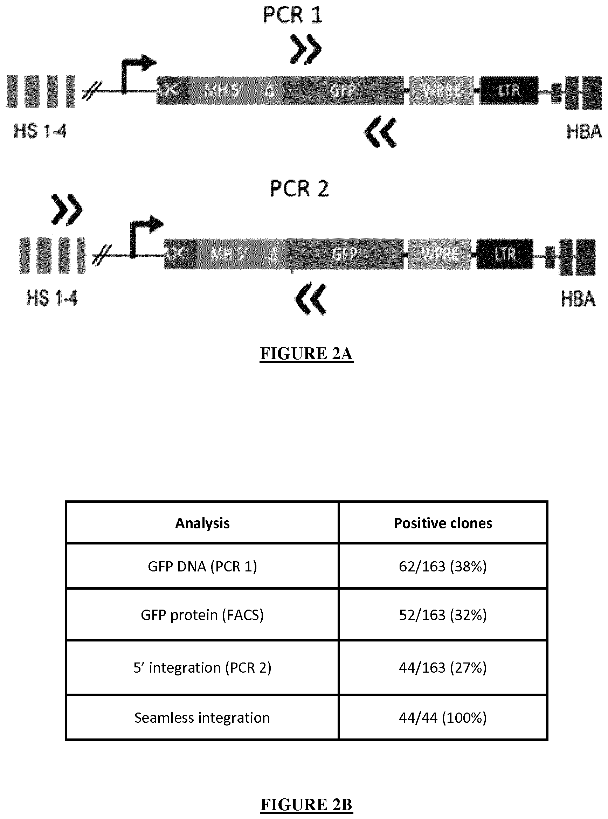

**Figure 6C****:** Summary table of DNA analyses of 13 CFU. To establish if the integration was seamless, the inventors performed Sanger sequencing of each PCR product coming from PCR 2 (as shown in Figure 2B).

### DETAILED DESCRIPTION OF THE INVENTION

As mentioned above, the inventors managed to generate an IDLV able to perform a precise and non-toxic sequence integration in the genome of a cell, with a high efficiency materialized by an efficient KI. Moreover, the inventors demonstrated that this IDLV even allows the integration of sequences having a substantial length.

In the nucleus of cells targeted with the IDLV of the invention, the lentiviral RNA genome is retrotranscribed to generate a dsDNA. Targeting this dsDNA with nucleases will generate free DNA ends that are more prone to interact with other DNA ends. Therefore, by generating, if in the same cells, a genomic DNA cut, the inventors managed to allow an increased KI of the sequence of interest.

### Integration-defective lentiviral vector

The present invention relates to an integration-defective lentiviral vector (IDLV) comprising a nucleic acid, the said nucleic acid comprising, between a 5' LTR sequence and a 3' LTR sequence:
a. at least one nucleus export signaling sequence, in particular at least one HIV-1 Rev Response Element (RRE), in particular one RRE,
b. at least one nucleic acid sequence of interest selected from the group consisting of a polyA signal; a splicing signal sequence; a DNA or RNA binding site; a promoter; and a transgene, or a fragment thereof, encoding a therapeutic protein or a therapeutic ribonucleic acid,
c. at least one nuclease site, in particular at least one guide nucleic acid targeted sequence (gRNA-T), and
d. at least one homology arm sequence consisting in a sequence which is homologous to a part of an endogenous genomic site of interest in the genome of a cell.

In the present text, an LTR designates a *"long terminal repeat",* well known by the man skilled in the art.

In the present text, the terms "upsteam" and "downsteam" relating to a sequence position compared to another sequence position is to be considered in the 5' to 3' direction: the upstream sequence is placed "before" the other sequence (which is thus the downstream sequence) when reading from 5' to 3'.

By placed before, in the absence of a contrary indication, it can mean either immediately or not immediately before.

Lentiviruses possess gag, pol, and env genes in addition to other accessory genes that are flanked by two long terminal repeat (LTR) sequences.

Like other lentiviruses, the IDLVs comprise a recombinant genome comprising, between the LTR 5' and 3' lentiviral sequences, a lentiviral encapsidation psi sequence, an RNA nuclear export element, a nucleic sequence of interest (for example a transgene), a splicing donor/acceptor site, and a cPPT sequence (Dull et al. (1998) J. Vir. 72:8463; Sirven et al. (2000) Blood, (96)203)). The designs of IDLV constructs have been described for example in Shaw et al. Biomedecines, 2014, 2, 14-35

The production of IDVL is similar to that of lentiviral vectors, which is well known in the state of the art. One skilled in the art may refer to general knowledge in this field, notably represented by Merten et al. (2016) Molecular therapy (3) 16017, Sharon and Kamen (2017) Biotechnology and Bioengineering (115) 25, Merten et al. (2011) Hum.Gene Ther 22(3) 343, Schweizer and Merten (2010) Cur.Gene Ther. 10(6) 474, Ansorgeet et al. (2010). Biochem. Eng. J. 48(3): 362. IDLVs may be produced for example using lentivirus vectors that include one or more mutations in the native lentivirus integrase gene itself or in the integrase recognition sequences in the viral LTR as disclosed in Yanez-Munoz et al. (2006) Nat Med 12(3):348-353; Nightingale et al. (2006) Mol Ther 13(6): 1121-1 132, WO2006/010834 and WO 2009/019612. Preferably, the IDLVs comprise a mutated integrase preventing the integration of said genome into the genome of a host cell. In a particular embodiment, the IDLVs carry a defective integrase with the mutation D64V in the catalytic domain of the enzyme.

An IDLV according to the invention can be pseudotyped, i.e. it comprises an envelope glycoprotein derived from a virus different from the virus from which is derived the IDLV, a modified envelope glycoprotein or a chimeric envelope glycoprotein. In a particular embodiment the IDLV is pseudotyped with a VSV-G, GALV-TR, RD114 or syncytin glycoprotein.

### Sequence a: nucleus export signaling sequence

As mentioned above, an IDLV of the invention is in particular characterized in that it comprises, between a 5' LTR sequence and a 3' LTR sequence, at least one nucleic acid comprising at least one nucleus export signaling sequence.

This at least one nucleus export signaling sequence can be present only once in an IDLV of the invention.

More particularly, this nucleus export signaling sequence can be upstream of the sequences b to e mentioned above.

Accordingly, in a particular embodiment, an IDLV of the invention is in particular characterized in that it comprises a nucleic acid comprising one nucleus export signaling sequence upstream of the sequences b to e mentioned above.

A nucleus export signaling sequence according to the invention can advantageously be a HIV-1 Rev Response Element (RRE).

The RRE sequence (REV Responsive Element) is a -350 nucleotide RNA sequence, in particular known for allowing export of viral messenger RNA from the nucleus to the cytosol after binding of the Rev protein, and thus as being essential for viral replication. It has however also been demonstrated that the presence of this element in lentiviral vectors is mandatory for efficient vector function (see for example Anson and Fuller; J. Gene Med. 2003; 5: 829-838).

In a particular embodiment of the invention, an IDLV of the invention is characterized in that it comprises a nucleic acid comprising one RRE sequence upstream of the sequences b to e mentioned above and described here-after.

### Sequence b: nucleic acid sequence of interest

As mentioned above, an IDLV of the invention is in particular characterized in that it comprises, between a 5' LTR sequence and a 3' LTR sequence, at least one nucleic acid comprising at least one nucleic acid sequence of interest.

This nucleic acid of interest is selected from the group consisting of a polyA signal; a splicing signal sequence; a DNA or RNA binding site; a promoter; and a transgene, or a fragment thereof, encoding a therapeutic protein or a therapeutic ribonucleic acid.

A polyA signal is generally an AAUAAA sequence, which induces a cleavage in the RNA at about 10-30 nucleotides downstream from said signal. Afterwards, a sequence of multiple adenosine monophosphates (AMP), i.e. a sequence that has only adenine bases. It can for example be constituted of from 50 to 300 adenosine monophosphates, in particular from 70 to 250 adenosine monophosphates.

A splicing signal sequence can be divided into those at the splice sites *per se* and auxiliary signals such as exonic splicing enhancers, intronic splicing enhancers and exonic splicing silencers. All splice sites conform to consensus sequences including nearly invariant dinucleotides at each end of the intron: GT at the 5' end of the intron, and AG at the 3' end of the intron, and generally have the following sequence MAG|GTRAGT at the 5' splice site and CAG|G at the 3' splice site.

A DNA or RNA binding site relates to, for example, a binding site for a transcriptional activator, a transcriptional repressor or an epigenetic modifier in order to modulate endogenous gene transcription. The RNA binding site can for example be an RNA sequence for binding protein in order to affect RNA stability, RNA localization, RNA nuclear export/import; a microRNA binding site for microRNA mediated transgene repression or knockdown.

A transgene according to the present invention encodes a therapeutic protein or a therapeutic ribonucleic acid.

In an embodiment of the invention, the said therapeutic protein or therapeutic ribonucleic acid can be any protein or ribonucleic acid providing a therapeutic effect to the cell into which the IDLV is present, in particular to the cell into which the sequence encoding the therapeutic protein or therapeutic ribonucleic acid is integrated into the endogenous genomic site of interest in the genome of the cell.

In another embodiment of the invention, the said therapeutic protein can be any protein providing a therapeutic effect outside of the cell into which the IDLV is present, in particular outside of the cell into which the sequence encoding the therapeutic protein or therapeutic ribonucleic acid is integrated into the endogenous genomic site of interest in the genome of the cell. The said therapeutic protein can indeed be secreted by the said cell or can be present, partially (for example a transmembrane protein) or completely on the surface of the said cell.

The therapeutic protein can in particular be selected from the group consisting of cytokines, in particular interferon, more particularly interferon-alpha, interferon-beta or interferon-pi; hormones; chemokines; antibodies (including nanobodies); anti-angiogenic factors; enzymes for replacement therapy, such as for example adenosine deaminase, alpha glucosidase, alpha-galactosidase, alpha-L-iduronidase and beta-glucosidase; interleukins; insulin; G-CSF; GM-CSF; hPG-CSF; M-CSF; blood clotting factors such as Factor VIII, Factor IX, tPA, Factor V, Factor VII, Factor X, Factor XI, Factor XII or Factor XIII; transmembrane proteins such as Nerve Growth Factor Receptor (NGFR); lysosomal enzymes such as α-galactosidase (GLA), α-L-iduronidase (IDUA), lysosomal acid lipase (LAL) and galactosamine (N-acetyl)-6-sulfatase (GALNS); beta-like globin; interleukin receptors such as IL-2 receptor, IL-3 receptor, IL-4 receptor, IL-5 receptor, IL-6 receptor, IL-7 receptor, IL-9 receptor, IL-11 receptor, IL-12 receptor, IL-13 receptor, IL-15 receptor, IL-21 receptor, IL-23 receptor and IL-27 receptor; Wiskott-Aldrich syndrome protein (WASP); adenosine deaminase, tripeptidyl peptidase 1, alpha-L iduronidase, iduronate 2-sulfatase, N-sulfoglucosamine sulfohydrolase, galactosamine-6 sulfatase, beta-galactosidase, N-acetylgalactosamine- 4-sulphatase, glucocerebrosidase, arylsulfatase A, cytochrome b-245 alpha chain, cytochrome b-245 beta chain, neutrophil cytosolic factor 1, neutrophil cytosolic factor 2, neutrophil cytosolic factor 4; any protein that can be engineered to be secreted and eventually uptaken by non-modified cells, and combinations thereof.

A transgene as described herein can for example encode at least one β-like globin protein and/or at least one β-like globin ribonucleic acid, and in particular encodes at least one functional β-like globin protein. A β-like globin gene according to the invention refers to a gene selected from the group consisting of epsilon-globin (ε), gamma-G-globin (G γ), gamma-A-globin (A γ), delta-globin (δ) and beta-globin (β) genes. In particular, a β-like globin gene according to the invention is a β-globin gene.

A transgene according to the invention can encode more than one therapeutic proteins and/or therapeutic ribonucleic acids. For example, a transgene according to the invention can encode two therapeutic proteins, in particular two different therapeutic proteins. Said therapeutic proteins can be as defined above. In another embodiment, a transgene according to the invention can encode two therapeutic ribonucleic acids, in particular two different therapeutic ribonucleic acids. Said therapeutic ribonucleic acid can be as defined above. In another embodiment, a transgene according to the invention can encode one therapeutic protein and one therapeutic ribonucleic acid, said therapeutic protein and therapeutic ribonucleic acid being in particular as defined above.

The invention also relates to fragments of a transgene of the invention as defined above. Said fragment can be a fraction of the therapeutic protein of interest having the same property as the said therapeutic protein, to a superior, similar or inferior intensity/level.

In a particular embodiment, the at least one nucleic acid sequence of interest is a transgene, or a fragment thereof, encoding a therapeutic protein or a therapeutic ribonucleic acid.

In a particular embodiment, an IDLV according to the invention comprises only one nucleic acid sequence of interest.

In another embodiment, an IDLV according to the invention comprises immediately before and/or immediately after its nucleic acid sequence(s) of interest at least one homology arm sequence (sequence d) as defined herein.

In a particular embodiment of the invention, an IDLV according to the invention comprises only one nucleic acid sequence of interest and at least one homology arm sequence present immediately before and/or immediately after the said nucleic acid sequence of interest.

In a particular embodiment, an IDLV according to the invention comprises two homology arm sequences, one homology arm sequence being upstream of the at least one, and in particular one, nucleic acid sequence of interest, the other homology arm sequence being downstream of the at least one, and in particular one, nucleic acid sequence of interest.

In another embodiment, an IDLV according to the invention comprises two homology arm sequences, the two homology arm sequences being upstream of the at least one, and in particular one, nucleic acid sequence of interest.

### Sequence c: nuclease site

As mentioned above, an IDLV of the invention is in particular characterized in that it comprises, between a 5' LTR sequence and a 3' LTR sequence, at least one nuclease site.

A nuclease site according to the invention is an RNA sequence which, once retrotranscribed into double stranded DNA, will be recognized by a nuclease. The nuclease will thus cleave the phosphodiester bonds between nucleotides of the nucleic acids. Depending of the nuclease, this action will be performed on a single or on both strands, leading to a single or double stranded break in the recognized DNA sequence.

Said nuclease site can for example be a site recognized by a guide peptide-containing endonuclease binding to a selected target site selected from a transcription activator-like effector nuclease (TALEN) or a zinc-finger nuclease.

The TALENs technology comprises a non-specific DNA-cleaving domain (nuclease) fused to a specific DNA-binding domain. The specific DNA-binding domain is composed of highly conserved repeats derived from transcription activator-like effectors (TALEs) which are proteins secreted by *Xanthomonas* bacteria to alter transcription of genes in host plant cells. The DNA-cleaving domain or cleavage half-domain can be obtained, for example, from various restriction endonucleases and/or homing endonucleases (for example Fok I Type IIS restriction endonuclease) of Fok I. (see Wright et al. (Biochem. J. 2014 Aug. 15;462(1): 15-24)).

The zinc-finger nuclease (ZFN) technology consists in the use of artificial restriction enzymes generated by fusion of a zinc finger DNA-binding domain to a DNA-cleavage domain (nuclease). The zinc finger domain specifically targets desired DNA sequences, which allows the associated nuclease to target a unique sequence within complex genomes.

The zinc finger DNA-binding domain comprises a chain of two-finger modules, each recognizing a unique hexamer (6bp) sequence of DNA. The two-finger modules are stitched together to form a Zinc finger protein. As in the TALENs technology, the DNA-cleavage domain comprises the nuclease domain of Fok I (Carroll D, Genetics, 2011 Aug; 188(4): 773-782; Urnov F.D. Nat Rev Genet. (9):636-46, (2010)).

In another embodiment, the nuclease site can be a site targeted by an endonuclease devoid of target site specificity, such as an RNA-guided endonuclease. Such RNA-guided endonuclease can in particular be a Clustered regularly interspaced short palindromic repeats (CRISPR) associated protein (Cas), in particular the CRISPR associated protein 9 (Cas9).

In this embodiment, the nuclease site is recognized by one or more guide nucleic acid, in particular guide RNA (gRNA). A nuclease site of the invention recognized by a guide nucleic acid, and in particular by a guide RNA, is also designated as being a guide nucleic acid targeted sequence (gRNA-T). This guide nucleic acid specifically targets and recognizes the nuclease site of the invention. The guide nucleic acid is also linked to an endonuclease devoid of target site specificity that will cleave the nuclease site of the IDLV as mentioned above.

Any gRNA can be used to target a nuclease site according to the invention, as long as it specifically targets it. For example, a gRNA that can be used according to the invention can in particular be one targeting a nuclease site within a globin gene, and more particularly one targeting a nuclease site present in any one of the genes selected from the group consisting of the epsilon globin gene, the gamma G globin gene, the gamma A globin gene, the delta globin gene, the beta globin gene, the zeta globin gene, the pseudozeta globin gene, the mu globin gene, the pseudoalpha-1 globin gene, the alpha 1 globin gene and the alpha 2 globin gene, and in particular selected from the group consisting of the gamma G globin gene, the gamma A globin gene, the delta globin gene, the beta globin gene, the alpha 1 globin gene and the alpha 2 globin gene, more particularly selected from the group consisting of the alpha 1 globin gene and the alpha 2 globin gene.

A gRNA as mentioned above can for example be selected from the group consisting of HBA-4, HBA-10, HBA-12, HBA-14, HBA 19.1, HBA 15.1, HBA 16.1, HBA 17-G, HBA 20.1, HBA 5.1, gRNA2, gRNA3, gRNA11, HBA INT1 72.1, HBA INT1 73.2, HBA INT1 73b.1, HBA INT2 13.2, HBA INT2 63.2, HBA INT2 74.1, HBB 37.1, HBB 49.2, HBB 53.1, HBB 54.1, HBB 77.1, HBB INT1 36.2, HBB INT1 36.2 REV, HBB INT1 47.1, HBB INT1 48.1, HBB INT2 340.1, HBB INT2 797.1, HBB INT2 20.1, HBB INT2 39.2, HBB KO et HBB AAVS1 targeting the sequence indicated in the following Table 1.

**Table 1**

| **HBA gRNA name** | **Partial targeted sequence** | **SEQ ID NO** | **Full targeted Sequence** | **SEQ ID NO** |
|---|---|---|---|---|
| HBA 4 | GGGGCGCGGCCTGGACCGCA | 1 | GGGGCGCGGCCTGGACCGCAGGG | 2 |
| HBA 10 | GGGTTTATGCTTGGGGCGCG | 3 | GGGTTTATGCTTGGGGCGCGGGG | 4 |
| HBA 12 | GACTCAGAGAGAACCCACCA | 5 | GACTCAGAGAGAACCCACCATGG | 6 |
| HBA 14 | TGGGTTCTCTCTGAGTCTGT | 7 | TGGGTTCTCTCTGAGTCTGTGGG | 8 |
| HBA 19.1 | GCGCGGGGGCACGCCCGGCC | 9 | GCGCGGGGGCACGCCCGGCCGGG | 10 |
| HBA 15.1 | GGGTTCTCTCTGAGTCTGTG | 11 | GGGTTCTCTCTGAGTCTGTGGGG | 12 |
| HBA 16.1 | GTCGGCAGGAGACAGCACCA | 13 | GTCGGCAGGAGACAGCACCATGG | 14 |
| HBA 17-G | GCAGGAGACAGCACCATGGT | 15 | GCAGGAGACAGCACCATGGTGGG | 16 |
| HBA 20.1 | CATAAACCCTGGCGCGCTCG | 17 | CATAAACCCTGGCGCGCTCGCGG | 18 |
| HBA 5.1 | TTGAATGCTCCAGCCGGTTC | 19 | TTGAATGCTCCAGCCGGTTCCAG | 20 |
| gRNA2 | CGGGAGGCTTCGCCCAATCC | 21 | CGGGAGGCTTCGCCCAATCCTGG | 22 |
| gRNA3 | CGGGCGAGCGAGTGCGAGCC | 23 | CGGGCGAGCGAGTGCGAGCCGG | 24 |
| gRNA11 | GGGAGGCTTCGCCCAATCCT | 25 | GGGAGGCTTCGCCCAATCCTGGG | 26 |
| HBA INT1 72 .1 | CAGGCCACCCTCAACCGTCC | 27 | CAGGCCACCCTCAACCGTCCTGG | 28 |
| HBA INT1 73 .2 | TCCGGGGCCAGGACGGTTGA | 29 | TCCGGGGCCAGGACGGTTGAGGG | 30 |
| HBA INT1 73b .1 | GTCCGGGGCCAGGACGGTTG | 31 | GTCCGGGGCCAGGACGGTTGAGG | 32 |
| HBA INT2 13.2 HBA | CCCTCGACCCAGATCGCTCC | 33 | CCCTCGACCCAGATCGCTCCCGG | 34 |
| INT2 63 .2 | GAAGAGGGTCAGTGCGGCCC | 35 | GAAGAGGGTCAGTGCGGCCCAGG | 36 |
| HBA INT2 74 .1 | GCGTGATCCTCTGCCCTGAG | 37 | GCGTGATCCTCTGCCCTGAGAGG | 38 |

| **HBB gRNA name** | **Partial targeted sequence** | **SEQ ID NO** | **Full targeted Sequence** | **SEQ ID NO** |
|---|---|---|---|---|
| HBB 37.1 | GGGTTGGCCAATCTACTCCC | 39 | GGTTGGCCAATCTACTCCCAGG | 40 |
| HBB 49.2 | GGGTTGGCCAATCTACTCCC | 41 | GGGTTGGCCAATCTACTCCCAGG | 42 |
| HBB 53.1 | GGAGTAGATTGGCCAACCCT | 43 | GGAGTAGATTGGCCAACCCTAGG | 44 |
| HBB 54.1 | GATTGGCCAACCCTAGGGTG | 45 | GATTGGCCAACCCTAGGGTGTGG | 46 |
| HBB 77.1 | GAGTAGATTGGCCAACCCTA | 47 | GAGTAGATTGGCCAACCCTAGGG | 48 |
| HBB INT 136.2 | TGGTATCAAGGTTACAAGAC | 49 | TGGTATCAAGGTTACAAGACAGG | 50 |
| HBB INT1 36.2 REV | TCCACATGCCCAGTTTCTAT | 51 | TCCACATGCCCAGTTTCTATTGG | 52 |
| HBB INT1 47.1 | TTAAGGAGACCAATAGAAAC | 53 | TTAAGGAGACCAATAGAAACTGG | 54 |
| HBB INT1 48.1 | TAAGGAGACCAATAGAAACT | 55 | TAAGGAGACCAATAGAAACTGGG | 56 |
| HBB INT2 340.1 | CTGCCTAGTACATTACTATT | 57 | CTGCCTAGTACATTACTATTTGG | 58 |
| HBB INT2 797 .1 | ATTAGCAAAAGGGCCTAGCT | 59 | ATTAGCAAAAGGGCCTAGCTTGG | 60 |
| HBB INT2 20.1 | GTTAAGTTCATGTCATAGGA | 61 | GTTAAGTTCATGTCATAGGAAGG | 62 |
| HBB INT2 39.2 | GACGAATGATTGCATCAGTG | 63 | GACGAATGATTGCATCAGTGTGG | 64 |
| HBB KO | CTTGCCCCACAGGGCAGTAA | 65 | CTTGCCCCACAGGGCAGTAACGG | 66 |
| HBB AAVS1 | GTCCCCTCCACCCCACAGTG | 67 | GTCCCCTCCACCCCACAGTG GGG | 68 |

An IDLV according to the invention in particular comprises, between its 5' LTR sequence and 3' LTR sequence, one or two nuclease site(s).

When two nuclease sites are present between the 5' LTR sequence and 3' LTR sequence of an IDLV of the invention, they can be different or identical. They are in particular different.

In a particular embodiment, an IDLV of the invention comprises at least two identical or different nuclease sites, in particular at least two identical or different gRNA-T sequences, and in particular two identical or different gRNA-T sequences.

According to this embodiment, an IDLV of the invention can be characterized in that it comprises at least one nucleic acid sequence of interest, which can be comprised between the at least two, and in particular two, nuclease sites of the said IDLV.

In an embodiment of the invention, the at least one nuclease site is identical to, or different from, an endogenous nuclease site comprised in the endogenous genomic site of interest in the genome of the cell of point d.

In a particular embodiment, between a 5' LTR sequence and a 3' LTR sequence of a nucleic acid comprised in an IDLV of the invention:
- when only one nuclease site is present, it is upstream of the at least one nucleic acid sequence of interest;
- when more than one nuclease sites, and in particular when two nuclease sites, are present, at least one, and in particular one, nuclease site is upstream of the at least one nucleic acid sequence of interest and at least one, in particular one, nuclease site is downstream of the at least one nucleic acid sequence of interest.

In a particular embodiment, between a 5' LTR sequence and a 3' LTR sequence of a nucleic acid comprised in an IDLV of the invention, when more than one nuclease sites, and in particular when two nuclease sites, are present, at least one, and in particular one, nuclease site is upstream of the at least one homology arm sequence and at least one, in particular one, nuclease site is downstream of the at least one homology arm sequence.

### Sequence d: homology arm

As previously mentioned, an IDLV of the invention comprises a nucleic acid comprising, between a 5' LTR sequence and a 3' LTR sequence, at least one homology arm sequence.

This at least one homology arm sequence consists in a sequence which is homologous to a part of an endogenous genomic site of interest in the genome of a cell, in particular in the genome of a cell into which the IDLV is intended to be integrated. An homology arm in an IDLV of the invention will in particular favor the directional Knock-In of the at least one nucleic acid sequence of interest into the endogenous genomic site of interest.

Such a cell into which the IDLV is intended to be integrated, and thus whose genome comprises an endogenous genomic site of interest, is further described in the present text and is also termed an isolated cell.

According to an embodiment, said cell can in particular be selected from the group consisting of hematopoietic stem cells; cells of the immune system, in particular lymphocytes; pluripotent stem cells; embryonic stem cells; satellite cells; neural stem cells; mesenchymal stem cells; retinal stem cells; and epithelial stem cells, and is in particular a hematopoietic stem cell.

An IDLV of the invention in particular comprises, between a 5' LTR sequence and a 3' LTR sequence, one or two homology arm sequences.

Exemplary homology arm lengths include a least 20, 30, 40, 50, 100, 250, 500, 750, 1000, 2000, 3000, 4000, or 5000 nucleotides. In some embodiments, the homology arm length is 50-100, 100-250, 250-500, 500-750, 750-1000, 1000-2000, 2000-3000, 3000-4000, or 4000-5000 nucleotides.

According to an embodiment, a homology arm sequence is of 40 nucleotides.

In a particular embodiment, the position of a homology arm sequence in an IDLV of the invention is immediately upstream (i.e. in 5') and/or immediately downstream (i.e. in 3') of the at least one nucleic acid sequence of interest.

Accordingly, in an embodiment of the invention, when an IDLV of the invention comprises, between a 5' LTR sequence and a 3' LTR sequence, one homology arm sequence, the said homology arm sequence is immediately upstream (i.e. in 5') and/or immediately downstream (i.e. in 3') of the at least one nucleic acid sequence of interest, and in particular of the one nucleic acid sequence of interest.

According to a particular embodiment, the IDLV comprises at least two homology arm sequences, in particular two, each one being different from the other and consisting in sequences which are homologous to at least two, in particular two, different parts of an endogenous genomic site of interest in the genome of the cell.

When an IDLV of the invention comprises, between a 5' LTR sequence and a 3' LTR sequence, two homology arm sequences, at least one of the said homology arm sequences is immediately upstream (i.e. in 5') and/or immediately downstream (i.e. in 3') of the at least one nucleic acid sequence of interest, and in particular of the one nucleic acid sequence of interest.

An endogenous genomic site of interest according to the invention may be an exon of a gene, an intron of a gene, a promoter, a 5'UTR region of a gene, a 3'UTR region of a gene, or an intergenic sequence.

It can in particular be a safe harbour.

Safe-harbours are chromosomal locations in a host genome where the at least one nucleic acid sequence of interest of the invention can integrate and function in a predictable manner without perturbing endogenous gene activity or promoting cancer (Sadelain et al., Nat Rev Cancer. 2011 Dec 1;12(1):51-8). Safe-harbours, also termed Genomic Safe-Harbours (GSHs), are intragenic or extragenic regions of the genome of a cell that are able to accommodate the predictable expression of newly integrated DNA without adverse effects on the host cell or organism. A useful safe-harbour must permit sufficient transgene expression to yield desired levels of the at least one nucleic acid sequence of interest of the invention.

As specific intragenic loci of the human genome that could be used as safe-harbour in a human cell can for example be mentioned the adeno-associated virus site 1 (AAVS1) (chromosome 19 position 19q13.42), the chemokine (CC motif) receptor 5 (CCR5) gene locus (chromosome 3 position 3q21.31) and the human orthologue of the mouse ROSA26 locus (chromosome 3 position 3q25.3).

In a particular embodiment, the endogenous genomic site of interest in the genome of the cell is comprised within a globin gene, in particular selected from the group consisting of the epsilon globin gene, the gamma G globin gene, the gamma A globin gene, the delta globin gene, the beta globin gene, the zeta globin gene, the pseudozeta globin gene, the mu globin gene, the pseudoalpha-1 globin gene, the alpha 1 globin gene and the alpha 2 globin gene, in particular selected from the group consisting of the gamma G globin gene, the gamma A globin gene, the delta globin gene, the beta globin gene, the alpha 1 globin gene and the alpha 2 globin gene, more particularly selected from the group consisting of the alpha 1 globin gene and the alpha 2 globin gene.

In a particular embodiment, an endogenous genomic site of interest according to the invention is in a gene intended to be completely or partially replaced by the at least one nucleic acid sequence of interest of the invention. Indeed, the transgene according to the invention can be integrated into its cognate locus, for example insertion of a wild type transgene into the endogenous locus, to correct a mutant gene.

In another embodiment, an endogenous genomic site of interest according to the invention is a T-cell receptor encoding gene in a T cell. According to this embodiment, the said endogenous genomic site of interest according to the invention can in particular encode a chimeric antigen receptor (CAR, also known as chimeric immunoreceptor, artificial T cell receptor or chimeric T cell receptor) which is able to target a target of interest while being able to activate the T-cell immunological function when binding to said target (antigen). The accordingly obtained CAR T cell (Chimeric antigen receptor T cell) is well known in the art, for the treatment of many diseases, and in particular in the treatment of cancers (see for example Raje et al. N. Engl. J. Med. 380; 18;1726-1737).

In another embodiment, an endogenous genomic site of interest according to the invention is an immunoglobulin encoding gene in a B cell. According to this embodiment, the said endogenous genomic site of interest according to the invention can in particular encode a therapeutic protein, and more particularly an immunoglobulin, or a fragment thereof, of interest (see Voss et al. eLife 2019;8:e42995 and T-C Cheong; Nat Commun. 2016 Mar 9;7:10934).

In a further embodiment, an endogenous genomic site of interest according to the invention is a T-cell receptor encoding gene or an immunoglobulin encoding gene in an Hematopoietic Stem Cell (HSC).

### Sequence e: sequence enhancing the stable expression of the nucleic acid sequence of interest (sequence b)

As previously mentioned, an IDLV of the invention can comprise, between a 5' LTR sequence and a 3' LTR sequence, at least one sequence which allows enhancing stable expression of the at least one nucleic acid sequence of interest.

In particular, the at least one sequence which allows enhancing stable expression of the at least one nucleic acid sequence of interest is a Woodchuck hepatitis virus Post-transcriptional Regulatory Element (WPRE) sequence.

The Woodchuck Hepatitis Virus (WHP) Posttranscriptional Regulatory Element (WPRE) is a sequence of 597 nucleotides or less (Schambach et al., Gene Ther. 2006 Apr;13(7):641-5) that, when transcribed, creates a tertiary structure enhancing expression. This sequence is commonly used in molecular biology to increase expression of genes delivered by viral vectors. WPRE is a tripartite regulatory element with gamma, alpha, and beta components. The alpha component is 80bp long and can be used alone, but is preferably used in combination with the gamma and beta components.

In a particular embodiment, an IDLV of the invention comprises only one sequence which allows enhancing stable expression of the at least one nucleic acid sequence of interest.

In a particular embodiment, an IDLV of the invention comprises only one Woodchuck hepatitis virus Post-transcriptional Regulatory Element (WPRE) sequence.

In a particular embodiment of the invention, an IDLV of the invention is in particular characterized in that, between the 5' LTR sequence and the 3' LTR sequence, the at least one sequence which allows enhancing stable expression of the at least one nucleic acid sequence of interest is downstream of the sequences a to d mentioned above.

In a particular embodiment of the invention, an IDLV of the invention is in particular characterized in that, between the 5' LTR sequence and the 3' LTR sequence, at least one, and in particular one, WPRE sequence is downstream of the sequences a to d mentioned above.

In a particular embodiment, sequences a to d, and e if present, as defined above are present in the IDLV, from 5' to 3', in one of the following orders:
- a, c, d, b;
- a, c, d, b, e;
- a, c, d, b, d, c;
- a, c, d, b, d, c, e;
- a, d, c, d, b;
- a, d, c, d, b, e;
- a, c, b, d, c, e; or
- a, c, d, b, c, e.

According to a particular embodiment, the IDLV according to the invention is circular or linear, and is in particular circular. More particularly, according to a particular embodiment, the intracellular form of an IDLV according to the invention is circular or linear, and is in particular circular.

According to another embodiment, the IDLV of the invention is linear. In particular, according to another embodiment, the intracellular form of an IDLV of the invention is linear.

According to a particular embodiment, the IDLV does not comprise a promotor.

### Isolated cells

As indicated above, the present invention further relates to an isolated cell comprising at least one IDLV as defined according to the invention.

According to a particular embodiment, an isolated cell is selected from the group consisting of hematopoietic stem cells (HSC); cells of the immune system, in particular lymphocytes; induced pluripotent stem cells; embryonic stem cells; satellite cells; neural stem cells; mesenchymal stem cells; retinal stem cells; and epithelial stem cells, and is in particular a hematopoietic stem cell.

In a particular embodiment of the invention, an isolated cell is different from an embryonic stem cell.

HSCs are pluripotent stem cells capable of self-renewal and are characterized by their ability to give rise under permissive conditions to all cell types of the hematopoietic system. HSC are not totipotent cells, i.e. they are not capable of developing into a complete organism.

In a particular embodiment, an HSC according to the invention is derived from an embryonic stem cell, in particular from a human embryonic stem cell, and is thus an embryonic hematopoietic stem cell.

Embryonic stem cells (ESCs) are stem cells derived from the undifferentiated inner mass cells of an embryo and capable of self-renewal. Under permissive conditions, these pluripotent stem cells are capable of differentiating in any one of the more than 220 cell types in the adult body. ESC are not totipotent cells, i.e. they are not capable of developing into a complete organism. ESC can for example be obtained according to the method indicated in Young Chung et al. (Cell Stem Cell 2, 2008 February 7;2(2): 113-7).

In another particular embodiment, an HSC according to the invention is an induced pluripotent stem cell, more particularly a human induced pluripotent stem cell (hiPSCs). Thus, according to a particular embodiment, HSC as described herein are hematopoietic induced pluripotent stem cells.
hiPSCs are genetically reprogrammed adult cells that exhibit a pluripotent stem cell-like state similar to ESC. They are artificially generated stem cells that are not known to exist in the human body but show qualities similar to those of ESC. Generating such cells is well known in the art as discussed in Ying WANG *et al*. (https://doi.org/10.1101/050021) as well as in Lapillonne H. et al. (Haematologica. 2010; 95(10)) and in J. DIAS et al. (Stem Cells Dev. 2011; 20(9): 1639-1647).

"Self renewal" refers to the ability of a cell to divide and generate at least one daughter cell with the identical (e.g., self-renewing) characteristics of the parent cell. The second daughter cell may commit to a particular differentiation pathway. For example, a self-renewing HSC can divide and form one daughter stem cell and another daughter cell committed to differentiation in the myeloid or lymphoid pathway. Self-renewal provides a continual source of undifferentiated stem cells for replenishment of the hematopoietic system.

The marker phenotypes useful for identifying HSCs will be those commonly known in the art. For human HSCs, the cell marker phenotypes preferably include any combination of CD34⁺ CD38^{low/-} Cd49f⁺ CD59⁺ CD90⁺ CD45RA⁻ Thyl⁺ C-kit⁺ lin⁻ (Notta F, Science. 333(6039):218-21 (2011)). For mouse HSCs, the cell marker phenotypes can illustratively be any combination of CD34^{low/-} Sca-1⁺ C-kit⁺ and lin⁻ CD150⁺ CD48⁻ CD90.1.Thy1^{+/low} Flk2/flt3⁻ and CD117⁺, (see, e.g., Frascoli et al. (J. Vis. Exp. 2012 Jul 8; (65). Pii:3736.).

Cells of the immune system are part of a host defense system comprising many biological structures and processes within an organism that protects against disease. According to the invention, cells of the immune system, include cells of the innate immune system and cells of the adaptive immune system.

Examples of cells of the innate immune system include leukocytes such as phagocytes (macrophages, neutrophils, and dendritic cells), innate lymphoid cells, mast cells, eosinophils, basophils, and natural killer cells.

Examples of cells of the adaptive immune system include lymphocytes, in particular B cells and T cells, such as killer T cells, Helper T cells.

According to a particular embodiment, the isolated cell according to the invention is a lymphocyte.

Satellite cells, also known as myosatellite cells or muscle stem cells are small multipotent cells with very little cytoplasm found in mature muscle. Satellite cells are precursors to skeletal muscle cells, able to give rise to satellite cells or differentiated skeletal muscle cells. They have the potential to provide additional myonuclei to their parent muscle fiber, or to return to a quiescent state. More specifically, upon activation, satellite cells can re-enter the cell cycle to proliferate and differentiate into myoblasts.

Neural stem cells (NSC) are self-renewing, multipotent cells that firstly generate the radial glial progenitor cells that generate the neurons and glia of the nervous system of all animals during embryonic development. Some neural progenitor stem cells persist in highly restricted regions in the adult vertebrate brain and continue to produce neurons throughout life.

Mesenchymal stem cells are multipotent stromal cells that can differentiate into a variety of cell types, including osteoblasts, chondrocytes, myocytes and adipocytes. As these cells are adult stem cells traditionally found in the bone marrow, they are also termed marrow stromal cells. They can however also be isolated from other tissues including cord blood, peripheral blood, fallopian tube, fetal liver and lung.

Retinal stem cells, also known as retinal progenitor cells, are pluripotent cells than can differentiate into the different cell types present in the retina.

Induced pluripotent stem cells (iPSC) are a type of pluripotent stem cell that can be generated directly from adult cells as mentioned above. Because they can propagate indefinitely, as well as give rise to every other cell type in the body (such as neurons, heart, pancreatic, and liver cells), they represent a single source of cells that could be used to replace those lost to damage or disease. Obtaining induced pluripotent stem cells belongs to the general knowledge of the man skilled in the art.

Epithelial stem cells play a central role in tissue homeostasis, wound repair, and carcinogenesis. Corneal epithelial stem cells have been demonstrated to reside in the limbal epithelium, while the fornical zone of the conjunctiva appears to be a predominant site of conjunctival epithelial stem cells. Stem cells of the corneal and conjunctival epithelia, as well as the hair follicle and interfollicular epidermis share important features: they are capable of self-renewal; they are relatively quiescent (slow-cycling); they can be induced to proliferate; and they are multipotent. It's becoming apparent that a certain degree of flexibility exists between corneal and hair follicle keratinocytes.

Isolated cells as described herein are preferably purified.

As used herein, *"purified cell"* means that the recited cells make up at least 50% of the cells in a purified sample; more preferably at least 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or more of the cells in a purified sample.

The cells' selection and/or purification can include both positive and negative selection methods to obtain a substantially pure population of cells.

In one aspect, fluorescence activated cell sorting (FACS), a flow cytometry technique, can be used to sort and analyze the different cell populations. Cells having specific cellular markers are tagged with an antibody, or typically a mixture of antibodies, that binds the cellular markers. Each antibody directed to a different marker is conjugated to a detectable molecule, particularly a fluorescent dye that can be distinguished from other fluorescent dyes coupled to other antibodies. A stream of stained cells is passed through a light source that excites the fluorochrome and the emission spectrum from the cells detects the presence of a particular labelled antibody. By concurrent detection of different fluorochromes, cells displaying different sets of cell markers are identified and isolated from other cells in the population. Other FACS parameters, including, by way of example and not limitation, side scatter (SSC), forward scatter (FSC), and vital dye staining (e.g., with propidium iodide) allow selection of cells based on size and viability.

In another aspect, immunomagnetic labelling can be used to sort the different cell population. This method is based on the attachment of small magnetizable particles to cells via antibodies or lectins. When the mixed population of cells is placed in a magnetic field, the cells that have beads attached will be attracted by the magnet and may thus be separated from the unlabeled cells.

According to a particular embodiment, HSCs that may be genetically modified according to the invention present a β-hemoglobinopathy phenotype, i.e. present a diminished expression of β-like globin compared to a healthy corresponding cell. In particular, the HSC cells that will be genetically modified according to the invention present a β-thalassemia or sickle-cell disease phenotype.

In a particular embodiment, an isolated cell, in particular an HSC, as described herein is a mammalian cell and in particular a human cell.

In a particular embodiment, the initial population of isolated cells may be autologous.

"Autologous" refers to deriving from or originating in the same patient or individual. An "autologous transplant" refers to the harvesting and reinfusion or transplant of a subject's own cells or organs. Exclusive or supplemental use of autologous cells can eliminate or reduce many adverse effects of administration of the cells back to the host, particular graft versus host reaction.

In this case, the isolated cells were collected from the said individual, genetically modified *ex vivo* or *in vitro* according to a method as described herein and administered to the same individual.

In another embodiment, the initial population of isolated cells may be derived from an allogeneic donor or from a plurality of allogeneic donors. The donors may be related or unrelated to each other, and in the transplant setting, related or unrelated to the recipient (or individual).

The isolated cells to be modified as described herein may accordingly be exogenous to the individual in need of therapy.

The isolated cells described herein may be resuspended in a pharmaceutically acceptable carrier and used directly or may be subjected to processing by various cell purification techniques available to the skilled artisan, such as FACS sorting, magnetic affinity separation, and immunoaffinity columns.

### Pharmaceutical composition

The present invention also relates to a pharmaceutical composition comprising at least one IDLV as described herein and/or at least one isolated cell as described herein, and a pharmaceutically acceptable medium.

A pharmaceutically acceptable medium as described herein is in particular suitable for administration to a mammalian individual.

A "pharmaceutically acceptable medium" comprises any of standard pharmaceutically accepted mediums known to those of ordinary skill in the art in formulating pharmaceutical compositions, for example, saline, phosphate buffer saline (PBS), aqueous ethanol, or solutions of glucose, mannitol, dextran, propylene glycol, oils (e.g., vegetable oils, animal oils, synthetic oils, etc.), microcrystalline cellulose, carboxym ethyl cellulose, hydroxylpropyl methyl cellulose, magnesium stearate, calcium phosphate, gelatine or polysorbate 80 or the like.

A pharmaceutical composition as described herein will often further comprise one or more buffers (e.g., neutral buffered saline or phosphate buffered saline); carbohydrates (e.g., glucose, mannose, sucrose or dextrans); mannitol; proteins; polypeptides or amino acids such as glycine; antioxidants (e.g., ascorbic acid, sodium metabisulfite, butylated hydroxytoluene, butylated hydroxyanisole, etc.); bacteriostats; chelating agents such as EDTA or glutathione; solutes that render the formulation isotonic, hypotonic or weakly hypertonic with the blood of a recipient; suspending agents; thickening agents and/or preservatives.

Of course, the type of carrier will typically vary depending on the mode of administration.

In an embodiment, the IDLV and/or isolated cells as described herein can be used in a composition in combination with therapeutic compounds that are effective in treating
- a disease selected from the group consisting of immune diseases, viral infections, tumors and blood diseases; and/or
- a disease caused by the lack of a protein or by the presence of an aberrant non-functional one in an individual in need thereof.

In an embodiment, the IDLV and/or isolated cells as described herein can be used in a composition as described herein in combination with IDLV different from the one of the present invention and/or with isolated cells not comprising an IDLV according to the invention.

In an embodiment, the IDLV and/ or isolated cells as described herein can be used in a composition of the invention in combination with other agents and compounds that enhance the therapeutic effect of the administered IDLV and/or cells.

### IDLV and isolated cells for their use as a medicament

Another object of the present invention is the IDLV or the isolated cell or the pharmaceutical composition defined above for use for its use as a medicament.

IDLVs, isolated cells and pharmaceutical compositions as described herein are administered into a subject by any suitable route, such as intravenous, intracardiac, intrathecal, intramuscular, intra-articular or intra-bone marrow injection, and in a sufficient amount to provide a therapeutic benefit.

The amount of IDLV or isolated cells needed for achieving a therapeutic effect will be determined empirically in accordance with conventional procedures for the particular purpose.

Generally, for administering the IDLV and/or isolated cells for therapeutic purposes, they are given at a pharmacologically effective dose.

By "pharmacologically effective amount" or "pharmacologically effective dose" is meant an amount sufficient to produce the desired physiological effect or amount capable of achieving the desired result, particularly for treating the disorder or disease condition, including reducing or eliminating one or more symptoms or manifestations of the disorder or disease.

Illustratively, administration of IDLV and/or isolated cells to a patient suffering from a β-thalassemia provides a therapeutic benefit when the amount of β-like globin, and therefore the amount of hemoglobin, in the patient is increased, when compared to the amount of β-like globin, and therefore the amount of hemoglobin, in the patient before administration.

IDLV and/or isolated cells are administered by methods well known in the art. In one embodiment, the administration is by intravenous infusion. In another method, the administration is by intra-bone marrow injection. In a further embodiment, the administration is by intra-ocular injection. In another embodiment, the administration is by intra-cerebral injection. In a further embodiment, the administration is by intramuscular injection.

The number of IDLVs and/or isolated cells transfused will take into consideration factors such as sex, age, weight, the types of disease or disorder, stage of the disorder, the percentage of the desired cells in the cell population (e.g., purity of cell population), and the cell number needed to produce a therapeutic benefit.

For instance, generally, the number of cells infused may be from 1.10⁴ to 5.10⁶ cells/kg, in particular from 1.10⁵ to 10.10⁶ cells/kg, preferably from 5.10⁵ cells to about 5.10⁶ cells/kg of body weight.

A pharmaceutical composition as described herein, as previously mentioned, can be used for administration of the IDLVs and/or isolated cells as described herein into the individual in need thereof.

The administration of IDLVs and/or isolated cells can be through a single administration or successive administrations. When successive administrations are involved, different cells numbers and/or different cells populations may be used for each administration.

Illustratively, a first administration can be of IDLVs and/or isolated cells as described herein that provides an immediate therapeutic benefit as well as more prolonged effect, while the second administration includes IDLVs and/or isolated cells as described herein that provide prolonged effect to extend the therapeutic effect of the first administration.

An IDLV, an isolated cell or a pharmaceutical composition as described herein can be used in the treatment of:
- a disease selected from the group consisting of immune diseases, viral infections, tumors and blood diseases; and/or
- a disease caused by the lack of a protein or by the presence of an aberrant non-functional one in an individual in need thereof.

In a particular embodiment, an IDLV, an isolated cell or a pharmaceutical composition as described herein can be used in the treatment of a disease caused by the lack of a secreted protein or by the presence of an aberrant non-functional secreted protein in an individual in need thereof.

Such disease can for example be selected from the group consisting a coagulation disorder, a lysosomal storage disorder, a hormonal defect and an alpha-1 antitrypsin deficiency.

An immune disease as mentioned above can for example be selected from the group consisting of 22q11.2 deletion syndrome; Adenosine Deaminase 2 deficiency; Adenosine deaminase deficiency; Adult-onset immunodeficiency with anti-interferon-gamma autoantibodies; Agammaglobulinemia, non-Bruton type; Aicardi-Goutieres syndrome ; Aicardi-Goutieres syndrome type 5; Allergic bronchopulmonary aspergillosis; Alopecia areata - Not a rare disease; Alopecia totalis; Alopecia universalis; Amyloidosis AA; Amyloidosis familial visceral; Ataxia telangiectasia; Autoimmune lymphoproliferative syndrome; Autoimmune lymphoproliferative syndrome due to CTLA4 haploinsuffiency; Autoimmune polyglandular syndrome type 1; Autosomal dominant hyper IgE syndrome; Autosomal recessive early-onset inflammatory bowel disease; Autosomal recessive hyper IgE syndrome ; Bare lymphocyte syndrome 2; Barth syndrome; Blau syndrome; Bloom syndrome; Bronchiolitis obliterans; C1q deficiency ; Candidiasis familial chronic mucocutaneous, autosomal recessive; Cartilage-hair hypoplasia; CHARGE syndrome; Chediak-Higashi syndrome; Cherubism; Chronic atypical neutrophilic dermatosis with lipodystrophy and elevated temperature; Chronic graft versus host disease; Chronic granulomatous disease; Cohen syndrome; Combined immunodeficiency with skin granulomas; Common variable immunodeficiency; Complement component 2 deficiency; Complement component 8 deficiency type 1; Complement component 8 deficiency type 2; Congenital pulmonary alveolar proteinosis; Cryoglobulinemia; Cutaneous mastocytoma; Cyclic neutropenia; Deficiency of interleukin-1 receptor antagonist; Dendritic cell, monocyte, B lymphocyte, and natural killer lymphocyte deficiency; Dyskeratosis congenita; Dyskeratosis congenita autosomal dominant; Dyskeratosis congenita autosomal recessive; Dyskeratosis congenita X-linked; Epidermodysplasia verruciformis; Familial amyloidosis, Finnish type; Familial cold autoinflammatory syndrome; Familial Mediterranean fever; Familial mixed cryoglobulinemia; Familiar chronic mucocutaneous candidiasis; Felty's syndrome; Glycogen storage disease type 1B; Griscelli syndrome type 2; Hashimoto encephalopathy; Hashimoto's syndrome; Hemophagocytic lymphohistiocytosis; Hennekam syndrome; Hepatic venoocclusive disease with immunodeficiency; Hereditary folate malabsorption; Hermansky Pudlak syndrome 2; Herpes simplex encephalitis; Hoyeraal Hreidarsson syndrome; Hyper IgE syndrome; Hyper-IgD syndrome; ICF syndrome; Idiopathic acute eosinophilic pneumonia; Idiopathic CD4 positive T-lymphocytopenia; IL12RB1 deficiency; Immune defect due to absence of thymus; Immune dysfunction with T-cell inactivation due to calcium entry defect 1; Immune dysfunction with T-cell inactivation due to calcium entry defect 2; Immunodeficiency with hyper IgM type 1; Immunodeficiency with hyper IgM type 2; Immunodeficiency with hyper IgM type 3; Immunodeficiency with hyper IgM type 4; Immunodeficiency with hyper IgM type 5; Immunodeficiency with thymoma; Immunodeficiency without anhidrotic ectodermal dysplasia; Immunodysregulation, polyendocrinopathy and enteropathy X-linked; Immunoglobulin A deficiency 2; Intestinal atresia multiple; IRAK-4 deficiency; Isolated growth hormone deficiency type 3; Kawasaki disease; Large granular lymphocyte leukemia; Leukocyte adhesion deficiency type 1; LRBA deficiency; Lupus; Lymphocytic hypophysitis; Majeed syndrome; Melkersson-Rosenthal syndrome; MHC class 1 deficiency; Muckle-Wells syndrome; Multifocal fibrosclerosis; Multiple sclerosis; MYD88 deficiency; Neonatal Onset Multisystem Inflammatory disease; Neonatal systemic lupus erythematosus; Netherton syndrome; Neutrophil-specific granule deficiency; Nijmegen breakage syndrome; Omenn syndrome; Osteopetrosis autosomal recessive 7; Palindromic rheumatism; Papillon Lefevre syndrome; Partial androgen insensitivity syndrome; PASLI disease; Pearson syndrome; Pediatric multiple sclerosis; Periodic fever, aphthous stomatitis, pharyngitis and adenitis; PGM3-CDG; Poikiloderma with neutropenia; Pruritic urticarial papules plaques of pregnancy; Purine nucleoside phosphorylase deficiency; Pyogenic arthritis, pyoderma gangrenosum and acne; Relapsing polychondritis; Reticular dysgenesis; Sarcoidosis; Say Barber Miller syndrome; Schimke immunoosseous dysplasia; Schnitzler syndrome; Selective IgA deficiency; Selective IgM deficiency; Severe combined immunodeficiency; Severe combined immunodeficiency due to complete RAG1/2 deficiency; Severe combined immunodeficiency with sensitivity to ionizing radiation; Severe combined immunodeficiency, atypical; Severe congenital neutropenia autosomal recessive 3; Severe congenital neutropenia X-linked; Short-limb skeletal dysplasia with severe combined immunodeficiency; Shwachman-Diamond syndrome; Singleton-Merten syndrome; SLC35C1-CDG (CDG-IIc); Specific antibody deficiency; Spondyloenchondrodysplasia; Stevens-Johnson syndrome/toxic epidermal necrolysis; T-cell immunodeficiency, congenital alopecia and nail dystrophy; TARP syndrome; Trichohepatoenteric syndrome; Tumor necrosis factor receptor-associated periodic syndrome; Twin to twin transfusion syndrome; Vici syndrome; WHIM syndrome; Wiskott Aldrich syndrome; Woods Black Norbury syndrome; X-linked agammaglobulinemia; X-linked immunodeficiency with magnesium defect, Epstein-Barr virus infection and neoplasia; X-linked lymphoproliferative syndrome; X-linked lymphoproliferative syndrome 1; X-linked lymphoproliferative syndrome 2; X-linked severe combined immunodeficiency and ZAP-70 deficiency.

A blood disease as mentioned above can for example be selected from the group consisting of 5q- syndrome; Aagenaes syndrome; Abdominal aortic aneurysm; Abetalipoproteinemia; Acatalasemia; Aceruloplasminemia; Acquired agranulocytosis; Acquired hemophilia; Acquired hemophilia A; Acquired pure red cell aplasia; Acquired Von Willebrand syndrome; Acute erythroid leukemia; Acute graft versus host disease; Acute monoblastic leukemia; Acute myeloblastic leukemia with maturation; Acute myeloblastic leukemia without maturation; Acute myeloid leukemia with abnormal bone marrow eosinophils inv(16)(p13q22) or t(16;16)(p13;q22); Acute myeloid leukemia with inv3(p21;q26.2) or t(3;3)(p21;q26.2); Acute myelomonocytic leukemia; Acute panmyelosis with myelofibrosis; Acute promyelocytic leukemia; Adenosine Deaminase 2 deficiency; Adrenocortical carcinoma; Adult T-cell leukemia/lymphoma; Afibrinogenemia; ALK+ histiocytosis; Alpha-thalassemia x-linked intellectual disability syndrome; AML with myelodysplasia-related features; Anemia due to Adenosine triphosphatase deficiency; Anemia sideroblastic and spinocerebellar ataxia; Aneurysm of sinus of Valsalva; Angioimmunoblastic T-cell lymphoma; Angioma hereditary neurocutaneous; Angioma serpiginosum; Antiphospholipid syndrome; Aplasia cutis congenita intestinal lymphangiectasia; Aplastic anemia; Arterial calcification of infancy; Arterial tortuosity syndrome; Atransferrinemia; Atypical hemolytic uremic syndrome; Autoimmune lymphoproliferative syndrome; Autosomal recessive protein C deficiency; Bannayan-Riley-Ruvalcaba syndrome; Behçet disease; Beta-thalassemia; Blastic plasmacytoid dendritic cell; Bleeding disorder due to P2RY12 defect; Bloom syndrome; Blue rubber bleb nevus syndrome; Buerger disease; Burkitt lymphoma; Campomelia Cumming type; Castleman disease; Cerebral cavernous malformation; Chediak-Higashi syndrome; Chromosome 17q11.2 deletion syndrome; Chronic myeloid leukemia; Chylous ascites; CLOVES syndrome; Cobb syndrome; Cold agglutinin disease; Congenital amegakaryocytic thrombocytopenia; Congenital analbuminemia; Congenital dyserythropoietic anemia type 1; Congenital dyserythropoietic anemia type 2; Congenital dyserythropoietic anemia type 3; Congenital erythropoietic porphyria; Congenital myasthenic syndrome with episodic apnea; Congenital pulmonary lymphangiectasia; Congenital thrombotic thrombocytopenic purpura; Cutaneous mastocytoma; Cutis laxa, autosomal recessive type 1; Cutis marmorata telangiectatica congenita; Cyclic neutropenia; Cyclic thrombocytopenia; Cystic medial necrosis of aorta; Dahlberg Borer Newcomer syndrome; Deafness-lymphedema-leukemia syndrome; Dehydrated hereditary stomatocytosis; Dehydrated hereditary stomatocytosis pseudohyperkalemia and perinatal edema; Diamond-Blackfan anemia; Diamond-Blackfan anemia 2; Diamond-Blackfan anemia 3; Dysfibrinogenemia; Dyskeratosis congenita; Dyskeratosis congenita autosomal dominant; Dyskeratosis congenita autosomal recessive; Dyskeratosis congenita X-linked; Ehlers-Danlos syndrome, dysfibronectinemic type; Eosinophilic granulomatosis with polyangiitis; Erythema elevatum diutinum; Essential thrombocythemia; Evans syndrome; Extranodal nasal NK/T cell lymphoma; Fabry disease; Factor V deficiency; Factor V Leiden thrombophilia; Factor VII deficiency; Factor X deficiency; Factor XI deficiency; Factor XII deficiency; Factor XIII deficiency; Familial hyperthyroidism due to mutations in TSH receptor; Familial LCAT deficiency; Familial platelet disorder with associated myeloid malignancy; Familial thoracic aortic aneurysm and dissection; Fanconi anemia; Fetal and neonatal alloimmune thrombocytopenia; Fibromuscular dysplasia; Follicular lymphoma; Genuine diffuse phlebectasia; Giant cell arteritis; Giant platelet syndrome; Glanzmann thrombasthenia; Glucocorticoid-remediable aldosteronism; Glutamate formiminotransferase deficiency; Glycogen storage disease type 12; Glycogen storage disease type 7; Glycoprotein VI deficiency; Goodpasture syndrome; Gorham's disease; Granulomatosis with polyangiitis; Granulomatous slack skin disease; Gray platelet syndrome; Hairy cell leukemia; Hashimoto-Pritzker syndrome; Heinz body anemias; Hemangioma thrombocytopenia syndrome; Hemochromatosis - Not a rare disease; Hemochromatosis type 2; Hemochromatosis type 3; Hemochromatosis type 4; Hemoglobin C disease; Hemoglobin E disease; Hemoglobin SC disease; Hemoglobin SE disease; Hemolytic anemia lethal congenital nonspherocytic with genital and other abnormalities; Hemolytic uremic syndrome; Hemophilia A; Hemophilia B; Hemorrhagic shock and encephalopathy syndrome; Hennekam syndrome; Henoch-Schonlein purpura; Heparin-induced thrombocytopenia; Hereditary antithrombin deficiency; Hereditary elliptocytosis; Hereditary folate malabsorption; Hereditary hemorrhagic telangiectasia; Hereditary hemorrhagic telangiectasia type 2; Hereditary hemorrhagic telangiectasia type 3; Hereditary hemorrhagic telangiectasia type 4; Hereditary lymphedema type II; Hereditary methemoglobinemia; Hereditary paraganglioma-pheochromocytoma; Hereditary spherocytosis; Hermansky Pudlak syndrome 2; High molecular weight kininogen deficiency; Histiocytosis-lymphadenopathy plus syndrome; Hoyeraal Hreidarsson syndrome; Hypercoagulability syndrome due to glycosylphosphatidylinositol deficiency; Hypereosinophilic syndrome; Hypersensitivity vasculitis; Hypocomplementemic urticarial vasculitis; Hypofibrinogenemia, familial; Hypotrichosis-lymphedema-telangiectasia syndrome; Idiopathic neutropenia; Idiopathic thrombocytopenic purpura; Imerslund-Grasbeck syndrome; Inclusion body myopathy 2; Inherited bone marrow failure syndromes; Internal carotid agenesis; Intrinsic factor deficiency; Iron-refractory iron deficiency anemia; Jacobsen syndrome; Juvenile myelomonocytic leukemia; Juvenile temporal arteritis; Kanzaki disease; Kaposi sarcoma; Kaposiform Hemangioendothelioma; Kaposiform lymphangiomatosis; Kawasaki disease; Klippel-Trenaunay syndrome; Langerhans cell sarcoma; Large granular lymphocyte leukemia; Lesch Nyhan syndrome; Liddle syndrome; Lipedema; Lissencephaly 2; Loeys-Dietz syndrome; Loeys-Dietz syndrome type 1; Loeys-Dietz syndrome type 2; Loeys-Dietz syndrome type 3; Loeys-Dietz syndrome type 4; Lymphedema and cerebral arteriovenous anomaly; Lymphedema-distichiasis syndrome; Lymphomatoid papulosis; Maffucci syndrome; Majeed syndrome; Mantle cell lymphoma; McLeod neuroacanthocytosis syndrome; Megalencephaly-capillary malformation syndrome; Megaloblastic anemia due to dihydrofolate reductase deficiency; Methemoglobinemia, beta-globin type; Methylcobalamin deficiency cbl G type; Methylmalonic acidemia and homocysteinemia type cblX; Methylmalonic acidemia with homocystinuria type cblC; Methylmalonic acidemia with homocystinuria type cblD; Methylmalonic acidemia with homocystinuria type cblF; Methylmalonic acidemia with homocystinuria type cblJ; Microcystic lymphatic malformation; Microscopic polyangiitis; Milroy disease; MPI-CDG (CDG-Ib); Multicentric Castleman Disease; Multifocal lymphangioendotheliomatosis with thrombocytopenia; Multiple myeloma; Multisystemic smooth muscle dysfunction syndrome; Myelodysplastic syndromes; Myelofibrosis; Myeloid sarcoma; MYH9 related thrombocytopenia; Neonatal hemochromatosis; Neutropenia chronic familial; Neutropenia lethal congenital with eosinophilia; Non-involuting congenital hemangioma; Nonspherocytic hemolytic anemia due to hexokinase deficiency; Noonan syndrome; Noonan syndrome 1; Noonan syndrome 2; Noonan syndrome 3; Noonan syndrome 4; Noonan syndrome 5; Noonan syndrome 6; Orotic aciduria type 1; Paris-Trousseau thrombocytopenia; Parkes Weber syndrome; Paroxysmal cold hemoglobinuria; Paroxysmal nocturnal hemoglobinuria; Pearson syndrome; PEHO syndrome; PHACE syndrome; Pheochromocytoma; Phosphoglycerate kinase deficiency; Plasmablastic lymphoma; Plasminogen activator inhibitor type 1 deficiency; Platelet storage pool deficiency; Plummer Vinson syndrome; POEMS syndrome; Poikiloderma with neutropenia; Polycythemia vera; Prekallikrein deficiency, congenital; Primary angiitis of the central nervous system; Primary central nervous system lymphoma; Primary familial and congenital polycythemia; Primary intestinal lymphangiectasia; Primary release disorder of platelets; Prolidase deficiency; Protein C deficiency; Protein S deficiency; Proteus syndrome; Prothrombin deficiency; Pseudo-Von Willebrand disease; Pseudohyperkalemia Cardiff; Pseudoxanthoma elasticum; Pulmonary arterio-veinous fistula; Pulmonary atresia with intact ventricular septum; Pulmonary vein stenosis; Purpura simplex; Pyropoikilocytosis hereditary; Pyruvate kinase deficiency; Quebec platelet disorder; Red cell phospholipid defect with hemolysis; Refractory cytopenia with unilineage dysplasia; Revesz syndrome; Reynolds syndrome; Rh deficiency syndrome; Rosai-Dorfman disease; Rotor syndrome; Scott syndrome; Severe congenital neutropenia autosomal dominant; Severe congenital neutropenia autosomal recessive 3; Sezary syndrome; Shwachman-Diamond syndrome; Sickle beta thalassemia; Sickle cell - hemoglobin D disease; Sickle cell anemia; Sideroblastic anemia; Sideroblastic anemia and mitochondrial myopathy; Sideroblastic anemia pyridoxine-refractory autosomal recessive; Sideroblastic anemia pyridoxine-responsive autosomal recessive; Slow-channel congenital myasthenic syndrome; Sneddon syndrome; Stomatocytosis I; Stomatocytosis II; Sturge-Weber syndrome; Supraumbilical midabdominal raphe and facial cavernous hemangiomas; Supravalvular aortic stenosis; Susac syndrome; Swyer syndrome; Systemic mastocytosis; T-cell/histiocyte rich large B cell lymphoma; Takayasu arteritis; TAR syndrome; Thalassemia; Thiamine responsive megaloblastic anemia syndrome; Thoracolaryngopelvic dysplasia; Thrombocytopathy asplenia miosis; Thrombocytopenia 2; Thrombocytopenia with elevated serum IgA and renal disease; Thrombomodulin anomalies, familial; Thrombotic thrombocytopenic purpura, acquired; Transient erythroblastopenia of childhood; Transient myeloproliferative syndrome; Triosephosphate isomerase deficiency; Tuberous sclerosis; Tufted angioma; Twin to twin transfusion syndrome; Type 1 plasminogen deficiency; Unicentric Castleman disease; Vascular Ehlers-Danlos syndrome; Vein of Galen aneurysm; Von Hippel-Lindau disease; Von Willebrand disease; Warm antibody hemolytic anemia; White platelet syndrome; Williams syndrome; Wiskott Aldrich syndrome; WT limb blood syndrome; Wyburn-Mason syndrome; X-linked sideroblastic anemia; X-linked thrombocytopenia and Yellow nail syndrome.

Lysosomal storage disorders can for example be selected from Gaucher's disease (glucocerebrosidase deficiency-gene name: GBA), Fabry's disease (α galactosidase deficiency-GLA), Hunter's disease (iduronate-2-sulfatase deficiency-IDS), Hurler's disease (alpha-L iduronidase deficiency-IDUA), and Niemann-Pick's disease (sphingomyelin phosphodiesterase 1 deficiency-SMPD1).

In a particular embodiment, a disease caused by the lack of a protein or by the presence of an aberrant non-functional one in an individual can be selected from the group consisting of hemophilia B, hemophilia A, Adenosine Deaminase deficiency, Beta-thalassemia, Sickle cell anemia, Wiskott Aldrich syndrome, X-linked agammaglobulinemia, Chronic granulomatous disease (CGD), Common variable immunodeficiency, X-linked SCID, ADA-deficient SCID, Ataxia telangiectasia, Omenn syndrome, Fanconi anemia, WHIM syndrome, Fabry disease, Wolman disease, Factor V deficiency, Factor V Leiden thrombophilia, Factor VII deficiency, Factor X deficiency, Factor XI deficiency, Factor XII deficiency, Factor XIII deficiency.

In another embodiment, an IDLV, an isolated cell or a pharmaceutical composition as described herein can be used in the treatment of a disease selected from the group consisting of immune diseases, viral infections, tumors and blood diseases.

Indeed, according to an embodiment of the invention, the therapeutic protein of the invention can be a therapeutic antibody that can be used for neutralization of target proteins, like bacterio-toxins, or proteins that directly cause disease (e.g. VEGF in macular degeneration) as well as highly selective killing of cells whose persistence and replication endanger the host (e.g. cancer cells, as well as certain immune cells in autoimmune diseases).

The present invention also relates to a method for preventing and/or treating of:
- a disease selected from the group consisting of immune diseases, viral infections, tumors and blood diseases; and/or
- a disease caused by the lack of a protein or by the presence of an aberrant non-functional one in an individual in need thereof;
comprising administering to an individual in need thereof at least an IDLV according to the invention.

The present invention also relates to a method for preventing and/or treating of:
- a disease selected from the group consisting of immune diseases, viral infections, tumors and blood diseases; and/or
- a disease caused by the lack of a protein or by the presence of an aberrant non-functional one in an individual in need thereof;
comprising administering to an individual in need thereof at least an isolated cell according to the invention.

The present invention also relates to a method for preventing and/or treating of:
- a disease selected from the group consisting of immune diseases, viral infections, tumors and blood diseases; and/or
- a disease caused by the lack of a protein or by the presence of an aberrant non-functional one in an individual in need thereof;
comprising administering to an individual in need thereof at least a pharmaceutical composition according to the invention.

The invention further relates to the use of an IDLV of the invention, an isolated cell of the invention, or a pharmaceutical composition of the invention for the manufacture of a medicine for preventing and/or treating:
- a disease selected from the group consisting of immune diseases, viral infections, tumors and blood diseases; and/or
- a disease caused by the lack of a protein or by the presence of an aberrant non-functional one in an individual in need thereof.

### Method for generating CAR-T cells according to the invention

A method for generating CAR-T cells is also provided according to the invention.

The present invention indeed further relates to a method for generating CAR-T cells comprising integrating, into a lymphocyte T cell, at least one IDLV as defined above, the said IDLV comprising, as at least one nucleic acid sequence of interest, a transgene encoding a chimeric antigen receptor targeting cancer cells.

The present invention also relates to a method for generating CAR-T cells comprising integrating, into a hematopoietic stem cell, at least one IDLV as defined above, and transforming the said hematopoietic stem cell into a lymphocyte T cell;
the said IDLV comprising, as at least one nucleic acid sequence of interest, a transgene encoding a chimeric antigen receptor targeting cancer cells.

Chimeric antigen receptor T cells (also known as CAR-T cells) are T cells that have been genetically engineered to produce an artificial T-cell receptor. Chimeric antigen receptors (CARs, also known as chimeric immunoreceptors, chimeric T cell receptors or artificial T cell receptors) are receptor proteins that have been engineered to give T cells the new ability to target a specific protein. The receptors are chimeric because they combine both antigen-binding and T-cell activating functions into a single receptor.

According to a particular embodiment, a chimeric antigen receptor targeting cancer cells according to the invention may be selected from those targeting tumor associated surface antigens such as CD10, CD19, CD20, CD22, CD33, Fms-like tyrosine kinase 3 (FLT-3, CD135), chondroitin sulfate proteoglycan 4 (CSPG4, melanoma-associated chondroitin sulfate proteoglycan), Epidermal growth factor receptor (EGFR), Her2neu, Her3, IGFR, CD133, IL3R, fibroblast activating protein (FAP), CDCP1, Derlinl, Tenascin, frizzled 1 -10, the vascular antigens VEGFR2 (KDR/FLK1), VEGFR3 (FLT4, CD309), PDGFR-a (CD140a), PDGFR-β (CD140b) Endoglin, CLEC14, Tem1-8, and Tie2. Further examples may include A33, CAM PATH -1 (CDw52), Carcinoembryonic antigen (CEA), Carboanhydrase IX (MN/CA IX), CD21, CD25, CD30, CD34, CD37, CD44v6, CD45, CD133, de2-7 EGFR, EGFRvlll, EpCAM, Ep-CAM, Folate-binding protein, G250, Fms-like tyrosine kinase 3 (FLT-3, CD135), c-Kit (CD1 17), CSF1 R (CD1 15), HLA-DR, IGFR, IL-2 receptor, IL3R, MCSP (Melanoma-associated cell surface chondroitin sulphate proteoglycane), Muc-1, Prostate-specific membrane antigen (PSMA), Prostate stem cell antigen (PSCA), Prostate specific antigen (PSA), and TAG-72. Examples of antigens expressed on the extracellular matrix of tumors are tenascin and the fibroblast activating protein (FAP).

The present invention also relates to a method for generating B cells expressing a therapeutic protein, and in particular an immunoglobulin, of interest, comprising integrating, into a lymphocyte B cell, at least one IDLV as defined above,
the said IDLV comprising, as at least one nucleic acid sequence of interest, a transgene encoding the therapeutic protein, and in particular the immunoglobulin or a fragment thereof, of interest.

The present invention also relates to a method for generating B cells expressing a therapeutic protein, and in particular an immunoglobulin, of interest, comprising integrating, into a hematopoietic stem cell, at least one IDLV as defined above, and transforming the said hematopoietic stem cell into a lymphocyte B cell;
the said IDLV comprising, as at least one nucleic acid sequence of interest, a transgene encoding the therapeutic protein, and in particular the immunoglobulin or a fragment thereof, of interest.

The present invention is further illustrated by, without in any way being limited to, the examples herein.

### EXAMPLES

### Example 1: GFP knock-in through IDLV transduction (IDLV GP33)

The inventors generated an IDLV (termed GP33) encoding for a promoterless GFP and inserted a gRNA targeted sequence (gRNA-T) in 5' of GFP to cut the IDVL, and a ∼40bp nucleotide short homologous sequence (micro homology, MH) which is homologous with the genomic site of interest (**Figure 1A**).

As mentioned above, these insertions aimed at increasing the "reactivity" of the IDLV DNA (ability to interact with other DNA ends generated by nuclease cutting genomic DNA) and favoring its directional KI thanks to the homology sequence (Nakade et al. Nat Commun. 2014 Nov 20;5:5560; Hisano et al. Sci Rep. 2015 Mar 5;5:8841; Sugawara and Nikaido Antimicrob Agents Chemother. 2014 Dec;58(12):7250-7). As genomic target, the inventors chose the 5'UTR of the alpha-globin locus in order to integrate GFP under the transcriptional control of the alpha-globin promoter. Since the IDLV-GFP was promoterless, it was expressed only if the IDLV was integrated at the intended locus and with the correct orientation (**Figure 1B**).

K562 (ATCC® CCL243) cells were maintained in k562 medium: RPMI 1640 medium, Gibco; 2 mM glutamine and 10% fetal bovine serum (Lonza); 10 mM HEPES, 1 mM sodium pyruvate, 100U/ml penicillin/streptomycin (LifeTechnologies).

A stable clone of K562-Cas9 was made by subcloning K562 cells transduced with a lentiviral vector (Addgene #52962) expressing spCas9 and a blasticidin resistance cassette.

6 x 10⁵ K562-Cas9 cells were transduced with the IDLV GP33 at a multiplicity of infection (MOI) of 50 in presence of 8µM polybrene. The trap was designed to be expressed upon integration in one of alpha-globin gene.

The inventors transduced K562 cells stably expressing SpCas9 (K562-Cas9 cells) with the described IDLV and then transfected them with a plasmid encoding for a gRNA targeting both the IDLV and the genomic DNA (HBA 15.1). As control, the inventors transfected transduced cells with a gRNA cutting only the 5'UTR of the alpha-globin locus (with a similar efficiency and at the same site as HBA 15.1), but not the IDLV (HBA 16.1).

24h after transduction, cells were washed with Phosphate-buffered saline (PBS) and 2 x 10⁵ cells transfected with 200 ng of HBA 15.1 or HBA 16.1 gRNA-containing vector (Addgene #53188) using NucleofectorAmaxa 4D with SF Cell Line 4D-Nucleofector Kit (Lonza).

Five days after nucleofection, DNA was extracted using QuickExtract™ DNA Extraction Solution. 5 µl of genomic DNA were used to amplify the region that spans the cutting site of each gRNA using KAPA2G Fast ReadyMix (Kapa Biosystem). After Sanger sequencing, the percentage of insertions and deletions (InDel) was calculated by TIDE webtool (tide.deskgen.com).

After 2 weeks, the inventors evaluated GFP expression by FACS and observed that the cutting of the IDLV DNA increased about 5 times the percentage of positive cells, indicating a more efficient GFP KI in the genomic DNA (**Figure 1** **C**).

The % of InDel correlates with the percentage of genomic alleles that have been cut by Cas9.

### Example 2: Efficiency and precision of IDLV KI

To confirm the efficiency and precision of IDLV KI, the inventors performed PCR analyses on genomic DNA extracted from K562-Cas9 single cells clones (**Figure 2A**).

163 single cell clones were obtained by serial dilution of HBA 15.1 IDLV cells treated as described in figure 1.

Genomic DNA was extracted after 2 weeks using "MagNA Pure 96 DNA and Viral NA Small Volume" Kit and quantified using NanoDrop 8000 Spectrophotometer by Thermo Fisher Scientificas.

For PCR 1, screening for the presence of integrated GFP cassette in the clones was done using TaqMan qPCR. qPCR for human albumin gene was used as DNA + control (Probe GFP, FAM: 5'CAACGAGAAGCGCGATCACATGGTC3' (SEQ ID NO: 69), Forward GFP: 5'AGTCCGCCCTGAGCAAAGA3' ((SEQ ID NO: 70), Reverse GFP: 5'GCGGTCACGAACTCCAGC3' (SEQ ID NO: 71); Probe ALB (VIC): 5'CCTGTCATGCCCACACAAATCTCTCC3' (SEQ ID NO: 72), Forward ALB: 5'GCTGTCATCTCTTGTGGGCTGT3' (SEQ ID NO: 73), Reverse ALB: 5'ACTCATGGGAGCTGCTGGTTC3'(SEQ ID NO: 74)).

To confirm proper 5'genomic DNA-IDLV junction, the inventors performed qualitative PCR (PCR 2) (KAPA2G Fast ReadyMix; Kapa Biosystem) using a genome-specific (TATCGCCAGAGGGAAAGGGA (SEQ ID NO: 75)) and a trap-specific primer (GAACTTCAGGGTCAGCTTGC (SEQ ID NO: 76)) to produce an amplicon of 823 bp.

PCR 2 products were Sanger sequenced and compared to the genomic sequence to evaluate for the presence of any mismatch.

84% of integrated donor DNA expressed GFP (52 out of 62 clones), of which 85% was inserted as cut IDLV (44 out of 52 clones). Thanks to the presence of the MH sequence, 100% of the cut IDLV integrated in a seamless way, with no nucleotide change as compared to the wild type genomic sequence. **Figure 2B** is a table summarizing the molecular analyses of the clones.

### Example 3: Optimal timing between IDLV transduction and nuclease cutting

The inventors then performed an additional experiment to find the optimal timing between IDLV transduction and nuclease cutting, which allowed the most efficient IDLV KI.

For this, the inventors transduced wild type K562 with GP33 IDLV (as in example 1) and, at different time points, transfected cells with *in vitro* preassembled Cas9/gRNA complex (RNP). At each of the indicated time points 2 x 10⁵ of transduced cells were washed and nucleofected with 18 µl of SF Cell Line 4D-Nucleofector Kit using NucleofectorAmaxa 4D (Lonza) and 2,75 µl of described HBA 15.1 or HBA 16.1 RNP. The assembly of the Cas9 with gRNA (HBA 15.1 or HBA 16.1) was done by mixing 30 µM of bacterial purified Cas9 protein and 45 µM of Synthego's synthetic gRNA guides from (ratio 1:1,5 respectively) using the appropriate volume of Buffer 10X for 10 minutes at room temperature.

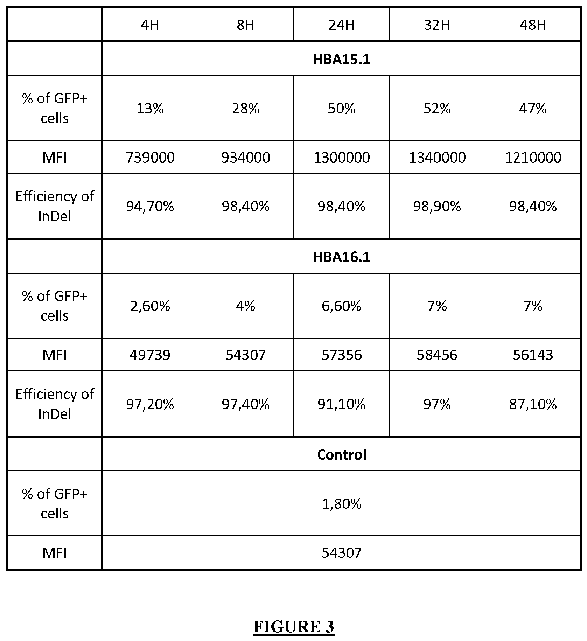

After 2 weeks, the inventors evaluated GFP expression by FACS and observed that a delay of 24-32 hours between IDLV and RNP delivery assured the best GFP expression, with the cut IDLV performing about 10 times better than the uncut IDLV (**Figure 3**). The higher efficiency of KI can be explained by the higher cutting performance of the RNP technology; in fact, the indel frequency for RNP results to be much higher than the efficiency obtained using gRNA plasmid (**Figure 1**).

### Example 4: BDD FVIII knock-in through IDLV transduction

Since one of the issues with current genome editing technology of HSC is the size of the DNA that can be integrated, the inventors decided to challenge their approach by inserting in the IDLV a big therapeutically relevant transgene. Therefore, they exchanged GFP (∼700 ntd) with the BDD FVIII coding sequence: coF8 (∼4400 ntd; ref.) (**Figure 4** **A**).

As for example 1, the inventors transduced K562-Cas9 with IDLV FVIII and then transfected them with a plasmid encoding for a gRNA targeting both the IDLV and the genomic DNA (HBA 15.1). To measure the efficiency and precision of IDLV KI, they performed PCR analyses on genomic DNA extracted from K562-Cas9 single cells clones (**Figure 4B**).

For PCR1, PCR screening for the presence of integrated coFVIII cassette was done using KAPA2G Fast ReadyMix (Kapa Biosystem) (Forward: GAGCTGTCCTGGGACTACAT (SEQ ID NO: 77), Reverse: GTGATCACCACGGTGTCGTA (SEQ ID NO: 78), amplicon 230bp).

To confirm proper 5'genomic DNA-IDLV junction, the inventors performed qualitative PCR (PCR 2) (KAPA2G Fast ReadyMix; Kapa Biosystem) using a genome-specific (TATCGCCAGAGGGAAAGGGA (SEQ ID NO: 79)) and coFVIII7R (GGGTTTTCTTGTACACCACGC (SEQ ID NO: 80)) primers, amplicon of 845bp.

82% of integrated donor DNA was inserted as cut IDLV (9 out of 11 clones). Thanks to presence of the MH sequence, 100% of the cut IDLV integrated in a seamless way, with no nucleotide change as compared to the wild type genomic sequence. **Figure 4C** is a table summarizing the molecular analyses of the clones.

After two weeks, the inventors analyzed FVIII expression by ELISA for the bulk population and 5 clones in the cells' supernatant (Asserachrom VIII:AG (Stago) ELISA kit). Since the IDLV FVIII is promoterless, expression was obtained only if: i) the IDLV was integrated at the intended locus, with the correct orientation and in a functional way; ii) K562 cells were capable of producing and secreting FVIII it is fully functional. FVIII production was expressed as the amount of FVIII produced in 24h by 2 x 10⁶ cells (**Figure 4** **D**).

Overall, this data shows that an IDLV according to the invention can efficiently deliver FVIII and integrate it under the control of the endogenous promoter. The produced FVIII is efficiently secreted by the cells and it maintains its enzymatic activity.

### Example 5: GFP knock-in through IDLV transduction (IDLV GP35)

The inventors further designed an IDLV (GP35) with gRNA-T in a different position to allow more flexibility in its design. To do so, they inserted an expression cassette containing in order, from 5' to 3': i) gRNA-T; ii) a promoterless GFP; iii) a PGK driven puromicin transgene. This cassette was inserted in antisense orientation relative to vector LTR As control, the inventors designed a similar IDLV missing the gRNA-T sequence as well as the MH5' sequence upstream the GFP (**Figure 5** **A**).

K562-Cas9 cells were transduced with these IDLV and, 24h later, transfected with HBA 15.1 gRNA encoding plasmid.

GFP expression analysis showed that once more the presence of the gRNA-T increased IDLV KI of almost 8 times as compared to control IDLV (**Figure 5** **B**).

The % of InDel correlates with the percentage of genomic alleles that have been cut by Cas9

### Example 6:

Finally, the inventors confirmed that the approach according to the invention could also be used to modify clinically relevant cells, such as CD34+ human HSPC.

The inventors cultured HSC for 48 hours, transduced them with GFP IDLV (**GP33**) and, 24 hours later, transfected them with RNP (HBA 15.1).

As control, the inventors used HBA 16.1 which only cut the genomic DNA, and not the IDLV. Cells were then differentiated towards the erythroid lineage to induce alpha-globin expression.

To do so, mobilized peripheral blood HSPC (AllCells) were thawed and cultured in prestimulation medium for 48 h (StemSpan, Stem Cell technologies; rhSCF 300 ng/ml, Flt3-L 300 ng/ml, rhTPO 100 ng/ml and IL-3 20 ng/mL, CellGenix). HSPC were transduced with IDLV GP33 on retronectin-coated plates (5µg/well), in the presence of protamine sulfate (4µg/ml) in prestimulation medium.

24 hours after transduction, cells were washed and electroporated with RNP (Cas9:gRNA HBA15.1; as in example 3). 2 x 10⁵ cells per condition were nucleofected with RNP complex using P3 Primary Cell 4D-Nucleofector kit (Lonza).

HSPC were cultured for 14 days in erythroid differentiation medium (StemSpan, Stem Cell Technologies; rhSCF 20 ng/ml, Epo 1 u/mL, IL3 5 ng/ml, Dexamethasone 2 µM and Betaestradiol 1 µM). GFP expression was monitored along differentiation by flow cytometry.

Although the cutting efficiency of both gRNA was similar (InDel efficiency), FACS analysis showed that nuclease targeted IDLV gave about 10 times more GFP positive cells than standard IDLV (**Figure 6A**).

The inventors plated HSPC in methylcellulose to obtain single cell colonies and monitor HSPC multipotency *in vitro.* After nucleofection, 10³ cells per condition were mixed with 3 ml of methylcellulose medium (H3434, StemCell Technologies) for colony-forming unit (CFC) assay.

After 14 days, colonies were counted and scored according to their appearance. Colonies were scored as granulocyte/erythrocyte/monocyte/megakaryocyte forming units (CFU-GEMM), granulocyte/macrophage forming units (CFU-GM) or erythroid burst-forming units (BFU-E).

Importantly, compared to the untreated control, the procedure of KI gave no toxicity or any lineage skewing to the progenitor cells (**Figure 6B**).

Single GFP positive colonies were picked under an inverted fluorescent microscope. DNA was extracted after lysis with proteinase K (Thermo Fisher Scientific) under standard conditions. Molecular analysis on genomic DNA was performed as for Example 2.

The inventors observed GFP expression in erythroid (BFU; 13 out of 99) but not in granulocyte/macrophage colonies, again indicating that GFP expression is driven by the erythroid specific alpha-globin promoter. To further confirm the precision of IDLV KI, they performed PCR analyses on genomic DNA extracted from GFP + single colonies to confirm the on-target donor DNA integration.

100% of inserted IDLV was cut and, thanks to presence of the MH sequence, 90 % of the cut IDLV integrated in a seamless way, with no nucleotide change as compared to the wild type genomic sequence (**Figure 6C**).

## Claims

1. An integration-defective lentiviral vector (IDLV) comprising a nucleic acid, the said nucleic acid comprising, between a 5' LTR sequence and a 3' LTR sequence:
a. at least one nucleus export signaling sequence, in particular at least one HIV-1 Rev Response Element (RRE), in particular one RRE;
b. at least one nucleic acid sequence of interest selected from the group consisting of a polyA signal; a splicing signal sequence; a DNA or RNA binding site; a promoter; and a transgene, or a fragment thereof, encoding a therapeutic protein or a therapeutic ribonucleic acid;
c. at least one nuclease site, in particular at least one guide nucleic acid targeted sequence (gRNA-T);
d. at least one homology arm sequence consisting in a sequence which is homologous to a part of an endogenous genomic site of interest in the genome of a cell; and
e. optionally, at least one sequence which allows enhancing stable expression of the at least one nucleic acid sequence of interest, in particular at least one Woodchuck hepatitis virus Post-transcriptional Regulatory Element (WPRE) sequence.

2. The IDLV according to claim 1, wherein the said IDLV is circular or linear, and is in particular circular.

3. The IDLV according to claim 1 or 2, comprising at least two identical or different nuclease sites, in particular at least two identical or different gRNA-T sequences, and in particular two identical or different gRNA-T sequences.

4. The IDLV according to claim 3, wherein the at least one nucleic acid sequence of interest is comprised between the at least two, and in particular two, nuclease sites of the said IDLV.

5. The IDLV according to any one of the preceding claims, wherein the at least one nuclease site is identical to, or different from, an endogenous nuclease site comprised in the endogenous genomic site of interest in the genome of the cell of point d.

6. The IDLV according to any one of the preceding claims, wherein the IDLV comprises at least two homology arm sequences, in particular two, each one being different from the other and consisting in sequences which are homologous to at least two, in particular two, different parts of an endogenous genomic site of interest in the genome of the cell.

7. The IDLV according to any one of the preceding claims, wherein the IDLV does not comprise a promotor.

8. The IDLV according to any one of the preceding claims, wherein the endogenous genomic site of interest in the genome of the cell is comprised within a globin gene, in particular selected from the group consisting of the epsilon globin gene, the gamma G globin gene, the gamma A globin gene, the delta globin gene, the beta globin gene, the zeta globin gene, the pseudozeta globin gene, the mu globin gene, the pseudoalpha-1 globin gene, the alpha 1 globin gene and the alpha 2 globin gene, in particular selected from the group consisting of the gamma G globin gene, the gamma A globin gene, the delta globin gene, the beta globin gene, the alpha 1 globin gene and the alpha 2 globin gene, more particularly selected from the group consisting of the alpha 1 globin gene and the alpha 2 globin gene.

9. The IDLV according to any one of the preceding claims, wherein the at least one nucleic acid sequence of interest is a transgene, or a fragment thereof, encoding a therapeutic protein or a therapeutic ribonucleic acid.

10. The IDLV according to claim 9, wherein the therapeutic protein is selected from the group consisting of cytokines, in particular interferon, more particularly interferon-alpha, interferon-beta or interferon-pi; hormones; chemokines; antibodies (including nanobodies); anti-angiogenic factors; enzymes for replacement therapy, such as for example adenosine deaminase, alpha glucosidase, alpha-galactosidase, alpha-L-iduronidase and beta-glucosidase; interleukins; insulin; G-CSF; GM-CSF; hPG-CSF; M-CSF; blood clotting factors such as Factor VIII, Factor IX, tPA, Factor V, Factor VII, Factor X, Factor XI, Factor XII or Factor XIII; transmembrane proteins such as Nerve Growth Factor Receptor (NGFR); lysosomal enzymes such as α-galactosidase (GLA), α-L-iduronidase (IDUA), lysosomal acid lipase (LAL) and galactosamine (N-acetyl)-6-sulfatase (GALNS); beta-like globin; interleukin receptors such as IL-2 receptor, IL-3 receptor, IL-4 receptor, IL-5 receptor, IL-6 receptor, IL-7 receptor, IL-9 receptor, IL-11 receptor, IL-12 receptor, IL-13 receptor, IL-15 receptor, IL-21 receptor, IL-23 receptor and IL-27 receptor; Wiskott-Aldrich syndrome protein (WASP); adenosine deaminase, tripeptidyl peptidase 1, alpha-L iduronidase, iduronate 2-sulfatase, N-sulfoglucosamine sulfohydrolase, galactosamine-6 sulfatase, beta-galactosidase, N-acetylgalactosamine- 4-sulphatase, glucocerebrosidase, arylsulfatase A, cytochrome b-245 alpha chain, cytochrome b-245 beta chain, neutrophil cytosolic factor 1, neutrophil cytosolic factor 2, neutrophil cytosolic factor 4; any protein that can be engineered to be secreted and eventually uptaken by non-modified cells, and combinations thereof.

11. The IDLV according to any one of the preceding claims, wherein sequences a to d, and e if present, are present in the IDLV, from 5' to 3', in one of the following orders:
- a, c, d, b;
- a, c, d, b, e;
- a, c, d, b, d, c;
- a, c, d, b, d, c, e;
- a, d, c, d, b;
- a, d, c, d, b, e;
- a, c, b, d, c, e; or
- a, c, d, b, c, e.

12. An isolated cell comprising at least one IDLV as defined in any one of claims 1 to 11.

13. The isolated cell according to claim 12, wherein the cell is selected from the group consisting of hematopoietic stem cells; cells of the immune system, in particular lymphocytes; pluripotent stem cells; embryonic stem cells; satellite cells; neural stem cells; mesenchymal stem cells; retinal stem cells; and epithelial stem cells, and is in particular a hematopoietic stem cell.

14. A pharmaceutical composition comprising at least one IDLV as defined in any one of claims 1 to 11 and/or at least one isolated cell as defined in claim 12 or 13, and a pharmaceutically acceptable medium.

15. The IDLV as defined in any one of claims 1 to 11, the isolated cell defined in claims 12 and 13 or the pharmaceutical composition defined in claim 14 for use in the treatment of:
- a disease selected from the group consisting of immune diseases, viral infections, tumors, and blood diseases; and/or
- a disease caused by the lack of a protein or by the presence of an aberrant non-functional one in an individual in need thereof.

16. Method for generating CAR-T cells comprising integrating, into a lymphocyte T cell or into a hematopoietic stem cell, at least one IDLV as defined in any one of claims 1 to 11, the said IDLV comprising, as at least one nucleic acid sequence of interest, a transgene encoding a chimeric antigen receptor targeting cancer cells.

17. Method for generating antibody expressing B-cells, comprising integrating, into a lymphocyte B cell or into a hematopoietic stem cell, at least one IDLV as defined in any one of claims 1 to 11,
the said IDLV comprising, as at least one nucleic acid sequence of interest, a transgene encoding an antibody.


SEQUENCE LISTING

<110> GENETHON
      UNIVERSITE D'EVRY-VAL-D'ESSONNE
      Institut National de la Sante et de la Recherche Medicale
      (INSERM)

<120> PRECISE INTEGRATION USING NUCLEASE TARGETED IDLV

<130> PR82368/MGG

<160> 80

<170> BiSSAP 1.3.6

<210> 1
<211> 20
<212> DNA
<213> Artificial Sequence

<220>
<223> Partial nucleic acid sequence of HBA 4

<220>
<223> Partial nucleic acid sequence of HBA 4

<400> 1
ggggcgcggc ctggaccgca                                                20

<210> 2
<211> 23
<212> DNA
<213> Artificial Sequence

<220>
<223> Full nucleic acid sequence of HBA 4

<220>
<223> Full nucleic acid sequence of HBA 4

<400> 2
ggggcgcggc ctggaccgca ggg                                            23

<210> 3
<211> 20
<212> DNA
<213> Artificial Sequence

<220>
<223> Partial nucleic acid sequence of HBA 10

<220>
<223> Partial nucleic acid sequence of HBA 10

<400> 3
gggtttatgc ttggggcgcg                                                20

<210> 4
<211> 23
<212> DNA
<213> Artificial Sequence

<220>
<223> Full nucleic acid sequence of HBA 10

<220>
<223> Full nucleic acid sequence of HBA 10

<400> 4
gggtttatgc ttggggcgcg ggg                                              23

<210> 5
<211> 20
<212> DNA
<213> Artificial Sequence

<220>
<223> Partial nucleic acid sequence of HBA 12

<220>
<223> Partial nucleic acid sequence of HBA 12

<400> 5
gactcagaga gaacccacca                                                  20

<210> 6
<211> 23
<212> DNA
<213> Artificial Sequence

<220>
<223> Full nucleic acid sequence of HBA 12

<220>
<223> Full nucleic acid sequence of HBA 12

<400> 6
gactcagaga gaacccacca tgg                                              23

<210> 7
<211> 20
<212> DNA
<213> Artificial Sequence

<220>
<223> Partial nucleic acid sequence of HBA 14

<220>
<223> Partial nucleic acid sequence of HBA 14

<400> 7
tgggttctct ctgagtctgt                                                  20

<210> 8
<211> 23
<212> DNA
<213> Artificial Sequence

<220>
<223> Full nucleic acid sequence of HBA 14

<220>
<223> Full nucleic acid sequence of HBA 14

<400> 8
tgggttctct ctgagtctgt ggg                                               23

<210> 9
<211> 20
<212> DNA
<213> Artificial Sequence

<220>
<223> Partial nucleic acid sequence of HBA 19.1

<220>
<223> Partial nucleic acid sequence of HBA 19.1

<400> 9
gcgcgggggc acgcccggcc                                                   20

<210> 10
<211> 23
<212> DNA
<213> Artificial Sequence

<220>
<223> Full nucleic acid sequence of HBA 19.1

<220>
<223> Full nucleic acid sequence of HBA 19.1

<400> 10
gcgcgggggc acgcccggcc ggg                                               23

<210> 11
<211> 20
<212> DNA
<213> Artificial Sequence

<220>
<223> Partial nucleic acid sequence of HBA 15.1

<220>
<223> Partial nucleic acid sequence of HBA 15.1

<400> 11
gggttctctc tgagtctgtg                                                   20

<210> 12
<211> 23
<212> DNA
<213> Artificial Sequence

<220>
<223> Full nucleic acid sequence of HBA 15.1

<220>
<223> Full nucleic acid sequence of HBA 15.1

<400> 12
gggttctctc tgagtctgtg ggg                                               23

<210> 13
<211> 20
<212> DNA
<213> Artificial Sequence

<220>
<223> Partial nucleic acid sequence of HBA 16.1

<220>
<223> Partial nucleic acid sequence of HBA 16.1

<400> 13
gtcggcagga gacagcacca                                                   20

<210> 14
<211> 23
<212> DNA
<213> Artificial Sequence

<220>
<223> Full nucleic acid sequence of HBA 16.1

<220>
<223> Full nucleic acid sequence of HBA 16.1

<400> 14
gtcggcagga gacagcacca tgg                                               23

<210> 15
<211> 20
<212> DNA
<213> Artificial Sequence

<220>
<223> Partial nucleic acid sequence of HBA 17-G

<220>
<223> Partial nucleic acid sequence of HBA 17-G

<400> 15

gcaggagaca gcaccatggt                                                    20

<210> 16
<211> 23
<212> DNA
<213> Artificial Sequence

<220>
<223> Full nucleic acid sequence of HBA 17-G

<220>
<223> Full nucleic acid sequence of HBA 17-G

<400> 16
gcaggagaca gcaccatggt ggg                                                23

<210> 17
<211> 20
<212> DNA
<213> Artificial Sequence

<220>
<223> Partial nucleic acid sequence of HBA 20.1

<220>
<223> Partial nucleic acid sequence of HBA 20.1

<400> 17
cataaaccct ggcgcgctcg                                                    20

<210> 18
<211> 23
<212> DNA
<213> Artificial Sequence

<220>
<223> Full nucleic acid sequence of HBA 20.1

<220>
<223> Full nucleic acid sequence of HBA 20.1

<400> 18
cataaaccct ggcgcgctcg cgg                                                23

<210> 19
<211> 20
<212> DNA
<213> Artificial Sequence

<220>
<223> Partial nucleic acid sequence of HBA 5.1

<220>
<223> Partial nucleic acid sequence of HBA 5.1

<400> 19
ttgaatgctc cagccggttc                                                    20

<210> 20
<211> 23
<212> DNA
<213> Artificial Sequence

<220>
<223> Full nucleic acid sequence of HBA 5.1

<220>
<223> Full nucleic acid sequence of HBA 5.1

<400> 20
ttgaatgctc cagccggttc cag                                                23

<210> 21
<211> 20
<212> DNA
<213> Artificial Sequence

<220>
<223> Partial nucleic acid sequence of gRNA2

<220>
<223> Partial nucleic acid sequence of gRNA2

<400> 21
cgggaggctt cgcccaatcc                                                    20

<210> 22
<211> 23
<212> DNA
<213> Artificial Sequence

<220>
<223> Full nucleic acid sequence of gRNA2

<220>
<223> Full nucleic acid sequence of gRNA2

<400> 22
cgggaggctt cgcccaatcc tgg                                                23

<210> 23
<211> 20
<212> DNA
<213> Artificial Sequence

<220>
<223> Partial nucleic acid sequence of gRNA3

<220>
<223> Partial nucleic acid sequence of gRNA3

<400> 23
cgggcgagcg agtgcgagcc                                                   20


<210> 24
<211> 22
<212> DNA
<213> Artificial Sequence


<220>
<223> Full nucleic acid sequence of gRNA3

<220>
<223> Full nucleic acid sequence of gRNA3

<400> 24
cgggcgagcg agtgcgagcc gg                                                22


<210> 25
<211> 20
<212> DNA
<213> Artificial Sequence


<220>
<223> Partial nucleic acid sequence of gRNA11

<220>
<223> Partial nucleic acid sequence of gRNA11

<400> 25
gggaggcttc gcccaatcct                                                   20


<210> 26
<211> 23
<212> DNA
<213> Artificial Sequence


<220>
<223> Full nucleic acid sequence of gRNA11

<220>
<223> Full nucleic acid sequence of gRNA11

<400> 26
gggaggcttc gcccaatcct ggg                                               23


<210> 27
<211> 20
<212> DNA
<213> Artificial Sequence


<220>
<223> Partial nucleic acid sequence of HBA INT1 72.1

<220>

<223> Partial nucleic acid sequence of HBA INT1 72.1

<400> 27
caggccaccc tcaaccgtcc                                                    20


<210> 28
<211> 23
<212> DNA
<213> Artificial Sequence


<220>
<223> Full nucleic acid sequence of HBA INT1 72.1

<220>
<223> Full nucleic acid sequence of HBA INT1 72.1

<400> 28
caggccaccc tcaaccgtcc tgg                                                23


<210> 29
<211> 20
<212> DNA
<213> Artificial Sequence


<220>
<223> Partial nucleic acid sequence of HBA INT1 73.2

<220>
<223> Partial nucleic acid sequence of HBA INT1 73.2

<400> 29
tccggggcca ggacggttga                                                    20


<210> 30
<211> 23
<212> DNA
<213> Artificial Sequence


<220>
<223> Full nucleic acid sequence of HBA INT1 73.2

<220>
<223> Full nucleic acid sequence of HBA INT1 73.2

<400> 30
tccggggcca ggacggttga ggg                                                23


<210> 31
<211> 20
<212> DNA
<213> Artificial Sequence


<220>
<223> Partial nucleic acid sequence of HBA INT1 73b.1

<220>
<223> Partial nucleic acid sequence of HBA INT1 73b.1

<400> 31
gtccggggcc aggacggttg                                                    20


<210> 32
<211> 23
<212> DNA
<213> Artificial Sequence


<220>
<223> Full nucleic acid sequence of HBA INT1 73b.1

<220>
<223> Full nucleic acid sequence of HBA INT1 73b.1

<400> 32
gtccggggcc aggacggttg agg                                                23


<210> 33
<211> 20
<212> DNA
<213> Artificial Sequence


<220>
<223> Partial nucleic acid sequence of HBA INT2 13.2

<220>
<223> Partial nucleic acid sequence of HBA INT2 13.2

<400> 33
ccctcgaccc agatcgctcc                                                    20


<210> 34
<211> 23
<212> DNA
<213> Artificial Sequence


<220>
<223> Full nucleic acid sequence of HBA INT2 13.2

<220>
<223> Full nucleic acid sequence of HBA INT2 13.2

<400> 34
ccctcgaccc agatcgctcc cgg                                                23


<210> 35
<211> 20
<212> DNA
<213> Artificial Sequence


<220>
<223> Partial nucleic acid sequence of HBA INT2 63.2

<220>
<223> Partial nucleic acid sequence of HBA INT2 63.2

<400> 35
gaagagggtc agtgcggccc                                                   20


<210> 36
<211> 23
<212> DNA
<213> Artificial Sequence


<220>
<223> Full nucleic acid sequence of HBA INT2 63.2

<220>
<223> Full nucleic acid sequence of HBA INT2 63.2

<400> 36
gaagagggtc agtgcggccc agg                                               23


<210> 37
<211> 20
<212> DNA
<213> Artificial Sequence


<220>
<223> Partial nucleic acid sequence of HBA INT2 74.1

<220>
<223> Partial nucleic acid sequence of HBA INT2 74.1

<400> 37
gcgtgatcct ctgccctgag                                                   20


<210> 38
<211> 23
<212> DNA
<213> Artificial Sequence


<220>
<223> Full nucleic acid sequence of HBA INT2 74.1

<220>
<223> Full nucleic acid sequence of HBA INT2 74.1

<400> 38
gcgtgatcct ctgccctgag agg                                               23


<210> 39
<211> 20
<212> DNA
<213> Artificial Sequence


<220>

<223> Partial nucleic acid sequence of HBB 37.1

<220>
<223> Partial nucleic acid sequence of HBB 37.1

<400> 39
gggttggcca atctactccc                                                    20


<210> 40
<211> 22
<212> DNA
<213> Artificial Sequence


<220>
<223> Full nucleic acid sequence of HBB 37.1

<220>
<223> Full nucleic acid sequence of HBB 37.1

<400> 40
ggttggccaa tctactccca gg                                                 22


<210> 41
<211> 20
<212> DNA
<213> Artificial Sequence


<220>
<223> Partial nucleic acid sequence of HBB 49.2

<220>
<223> Partial nucleic acid sequence of HBB 49.2

<400> 41
gggttggcca atctactccc                                                    20


<210> 42
<211> 23
<212> DNA
<213> Artificial Sequence


<220>
<223> Full nucleic acid sequence of HBB 49.2

<220>
<223> Full nucleic acid sequence of HBB 49.2

<400> 42
gggttggcca atctactccc agg                                                23


<210> 43
<211> 20
<212> DNA
<213> Artificial Sequence

<220>
<223> Partial nucleic acid sequence of HBB 53.1

<220>
<223> Partial nucleic acid sequence of HBB 53.1

<400> 43
   ggagtagatt ggccaaccct                                                  20


<210> 44
<211> 23
<212> DNA
<213> Artificial Sequence


<220>
<223> Full nucleic acid sequence of HBB 53.1

<220>
<223> Full nucleic acid sequence of HBB 53.1

<400> 44
   ggagtagatt ggccaaccct agg                                              23


<210> 45
<211> 20
<212> DNA
<213> Artificial Sequence


<220>
<223> Partial nucleic acid sequence of HBB 54.1

<220>
<223> Partial nucleic acid sequence of HBB 54.1

<400> 45
gattggccaa ccctagggtg                                                   20


<210> 46
<211> 23
<212> DNA
<213> Artificial Sequence


<220>
<223> Full nucleic acid sequence of HBB 54.1

<220>
<223> Full nucleic acid sequence of HBB 54.1

<400> 46
gattggccaa ccctagggtg tgg                                               23


<210> 47
<211> 20
<212> DNA
<213> Artificial Sequence

<220>
<223> Partial nucleic acid sequence of HBB 77.1

<220>
<223> Partial nucleic acid sequence of HBB 77.1

<400> 47
gagtagattg gccaacccta                                                   20


<210> 48
<211> 23
<212> DNA
<213> Artificial Sequence


<220>
<223> Full nucleic acid sequence of HBB 77.1

<220>
<223> Full nucleic acid sequence of HBB 77.1

<400> 48
gagtagattg gccaacccta ggg                                               23


<210> 49
<211> 20
<212> DNA
<213> Artificial Sequence


<220>
<223> Partial nucleic acid sequence of HBB INT 1 36.2

<220>
<223> Partial nucleic acid sequence of HBB INT 1 36.2

<400> 49
tggtatcaag gttacaagac                                                   20


<210> 50
<211> 23
<212> DNA
<213> Artificial Sequence


<220>
<223> Full nucleic acid sequence of HBB INT 1 36.2

<220>
<223> Full nucleic acid sequence of HBB INT 1 36.2

<400> 50
tggtatcaag gttacaagac agg                                               23


<210> 51
<211> 20
<212> DNA
<213> Artificial Sequence

<220>
<223> Partial nucleic acid sequence of HBB INT1 36.2 REV

<220>
<223> Partial nucleic acid sequence of HBB INT1 36.2 REV

<400> 51
tccacatgcc cagtttctat                                                   20


<210> 52
<211> 23
<212> DNA
<213> Artificial Sequence


<220>
<223> Full nucleic acid sequence of HBB INT1 36.2 REV

<220>
<223> Full nucleic acid sequence of HBB INT1 36.2 REV

<400> 52
tccacatgcc cagtttctat tgg                                               23


<210> 53
<211> 20
<212> DNA
<213> Artificial Sequence


<220>
<223> Partial nucleic acid sequence of HBB INT1 47.1

<220>
<223> Partial nucleic acid sequence of HBB INT1 47.1

<400> 53
ttaaggagac caatagaaac                                                   20


<210> 54
<211> 23
<212> DNA
<213> Artificial Sequence


<220>
<223> Full nucleic acid sequence of HBB INT1 47.1

<220>
<223> Full nucleic acid sequence of HBB INT1 47.1

<400> 54
ttaaggagac caatagaaac tgg                                               23


<210> 55
<211> 20
<212> DNA

<213> Artificial Sequence

<220>
<223> Partial nucleic acid sequence of HBB INT1 48.1

<220>
<223> Partial nucleic acid sequence of HBB INT1 48.1

<400> 55
taaggagacc aatagaaact                                                   20

<210> 56
<211> 23
<212> DNA
<213> Artificial Sequence

<220>
<223> Full nucleic acid sequence of HBB INT1 48.1

<220>
<223> Full nucleic acid sequence of HBB INT1 48.1

<400> 56
taaggagacc aatagaaact ggg                                               23

<210> 57
<211> 20
<212> DNA
<213> Artificial Sequence

<220>
<223> Partial nucleic acid sequence of HBB INT2 340.1

<220>
<223> Partial nucleic acid sequence of HBB INT2 340.1

<400> 57
ctgcctagta cattactatt                                                   20

<210> 58
<211> 23
<212> DNA
<213> Artificial Sequence

<220>
<223> Full nucleic acid sequence of HBB INT2 340.1

<220>
<223> Full nucleic acid sequence of HBB INT2 340.1

<400> 58
ctgcctagta cattactatt tgg                                               23

<210> 59
<211> 20

<212> DNA
<213> Artificial Sequence

<220>
<223> Partial nucleic acid sequence of HBB INT2 797.1

<220>
<223> Partial nucleic acid sequence of HBB INT2 797.1

<400> 59
attagcaaaa gggcctagct                                                   20

<210> 60
<211> 23
<212> DNA
<213> Artificial Sequence

<220>
<223> Full nucleic acid sequence of HBB INT2 797.1

<220>
<223> Full nucleic acid sequence of HBB INT2 797.1

<400> 60
attagcaaaa gggcctagct tgg                                               23

<210> 61
<211> 20
<212> DNA
<213> Artificial Sequence

<220>
<223> Partial nucleic acid sequence of HBB INT2 20.1

<220>
<223> Partial nucleic acid sequence of HBB INT2 20.1

<400> 61
gttaagttca tgtcatagga                                                   20

<210> 62
<211> 23
<212> DNA
<213> Artificial Sequence

<220>
<223> Full nucleic acid sequence of HBB INT2 20.1

<220>
<223> Full nucleic acid sequence of HBB INT2 20.1

<400> 62
gttaagttca tgtcatagga agg                                               23

<210> 63

<211> 20
<212> DNA
<213> Artificial Sequence

<220>
<223> Partial nucleic acid sequence of HBB INT2 39.2

<220>
<223> Partial nucleic acid sequence of HBB INT2 39.2

<400> 63
gacgaatgat tgcatcagtg                                                   20

<210> 64
<211> 23
<212> DNA
<213> Artificial Sequence

<220>
<223> Full nucleic acid sequence of HBB INT2 39.2

<220>
<223> Full nucleic acid sequence of HBB INT2 39.2

<400> 64
gacgaatgat tgcatcagtg tgg                                               23

<210> 65
<211> 20
<212> DNA
<213> Artificial Sequence

<220>
<223> Partial nucleic acid sequence of an HBB gRNA

<400> 65
cttgccccac agggcagtaa                                                   20

<210> 66
<211> 23
<212> DNA
<213> Artificial Sequence

<220>
<223> Full nucleic acid sequence of a HBB gRNA

<400> 66
cttgccccac agggcagtaa cgg                                               23

<210> 67
<211> 20
<212> DNA
<213> Artificial Sequence

<220>
<223> partial nucleic acid sequence of an AAVS1 gRNA

<400> 67
gtcccctcca ccccacagtg                                                    20


<210> 68
<211> 23
<212> DNA
<213> Artificial Sequence


<220>
<223> Full nucleic acid sequence of a AAVS1 gRNA

<400> 68
gtcccctcca ccccacagtg ggg                                                23


<210> 69
<211> 25
<212> DNA
<213> Artificial Sequence


<220>
<223> Probe GFP

<400> 69
caacgagaag cgcgatcaca tggtc                                              25


<210> 70
<211> 19
<212> DNA
<213> Artificial Sequence


<220>
<223> Forward GFP

<400> 70
agtccgccct gagcaaaga                                                     19


<210> 71
<211> 18
<212> DNA
<213> Artificial Sequence


<220>
<223> Reverse GFP

<400> 71
gcggtcacga actccagc                                                      18


<210> 72
<211> 26
<212> DNA
<213> Artificial Sequence

<220>
<223> Probe ALB (VIC)

<400> 72
cctgtcatgc ccacacaaat ctctcc                                          26


<210> 73
<211> 22
<212> DNA
<213> Artificial Sequence


<220>
<223> Forward ALB

<400> 73
gctgtcatct cttgtgggct gt                                              22


<210> 74
<211> 21
<212> DNA
<213> Artificial Sequence


<220>
<223> Reverse ALB

<400> 74
actcatggga gctgctggtt c                                               21


<210> 75
<211> 20
<212> DNA
<213> Artificial Sequence


<220>
<223> Genome-specific primer to confirm proper 5genomic DNA-IDLV
      junction

<400> 75
tatcgccaga gggaaaggga                                                 20


<210> 76
<211> 20
<212> DNA
<213> Artificial Sequence


<220>
<223> Trap-specific primer to confirm proper 5genomic DNA-IDLV
      junction

<400> 76
gaacttcagg gtcagcttgc                                                 20

<210> 77
<211> 20
<212> DNA
<213> Artificial Sequence

<220>
<223> Forward (PCR1) for screening for the presence of integrated
      coFVIII cassette

<400> 77
gagctgtcct gggactacat                                                   20

<210> 78
<211> 20
<212> DNA
<213> Artificial Sequence

<220>
<223> Reverse (PCR1) for screening for the presence of integrated
      coFVIII cassette

<400> 78
gtgatcacca cggtgtcgta                                                   20

<210> 79
<211> 20
<212> DNA
<213> Artificial Sequence

<220>
<223> Genome-specific primer to confirm proper 5genomic DNA-IDLV
      junction

<400> 79
tatcgccaga gggaaaggga                                                   20

<210> 80
<211> 21
<212> DNA
<213> Artificial Sequence

<220>
<223> coFVIII7R primer to confirm proper 5genomic DNA-IDLV junction

<400> 80
gggttttctt gtacaccacg c                                                 21